# EUROPEAN PATENT APPLICATION

(11) **EP 4 194 458 A1**
(43) Date of publication of application: **14.06.2023**
(21) Application number: 21856106.6
(22) Date of filing: 29.07.2021
(51) Int. Cl.: C07D 471/14, H01L 51/00, H01L 51/50

(54) **HETEROCYCLIC COMPOUND AND ORGANIC LIGHT-EMITTING DEVICE COMPRISING SAME**

(30) Priority: 10.08.2020 KR 20200099811
(71) Applicant: LT Materials Co., Ltd., Yongin-si, Gyeonggi-do 17118 (KR)
(72) Inventor: LEE, Gi-Back, Yongin-si, Gyeonggi-do 17118 (KR); LA, Hyun-Ju, Yongin-si, Gyeonggi-do 17118 (KR); JEONG, Won-Jang, Yongin-si, Gyeonggi-do 17118 (KR); KIM, Dong-Jun, Yongin-si, Gyeonggi-do 17118 (KR)
(74) Representative: Goddar, Heinz J.
(86) International application number: PCT/KR2021/009890
(87) International publication number: WO 2022/035100

(57) **Abstract**

The present specification relates to a heterocyclic compound represented by Chemical Formula 1, and an organic light emitting device including the same.

## Description

### [Technical Field]

The present specification relates to a heterocyclic compound, and an organic light emitting device including the same.

This application claims priority to and the benefits of Korean Patent Application No. 10-2020-0099811, filed with the Korean Intellectual Property Office on August 10, 2020, the entire contents of which are incorporated herein by reference.

### [Background Art]

An electroluminescent device is one type of self-emissive display devices, and has an advantage of having a wide viewing angle, and a high response speed as well as having an excellent contrast.

An organic light emitting device has a structure disposing an organic thin film between two electrodes. When a voltage is applied to an organic light emitting device having such a structure, electrons and holes injected from the two electrodes bind and pair in the organic thin film, and light emits as these annihilate. The organic thin film may be formed in a single layer or a multilayer as necessary.

A material of the organic thin film may have a light emitting function as necessary. For example, as a material of the organic thin film, compounds capable of forming a light emitting layer themselves alone may be used, or compounds capable of performing a role of a host or a dopant of a host-dopant-based light emitting layer may also be used. In addition thereto, compounds capable of performing roles of hole injection, hole transfer, electron blocking, hole blocking, electron transfer, electron injection and the like may also be used as a material of the organic thin film.

Development of an organic thin film material has been continuously required for enhancing performance, lifetime or efficiency of an organic light emitting device.

### [Disclosure]

### [Technical Problem]

The present specification is directed to providing a heterocyclic compound, and an organic light emitting device including the same.

### [Technical Solution]

One embodiment of the present specification provides a heterocyclic compound of the following Chemical Formula 1.

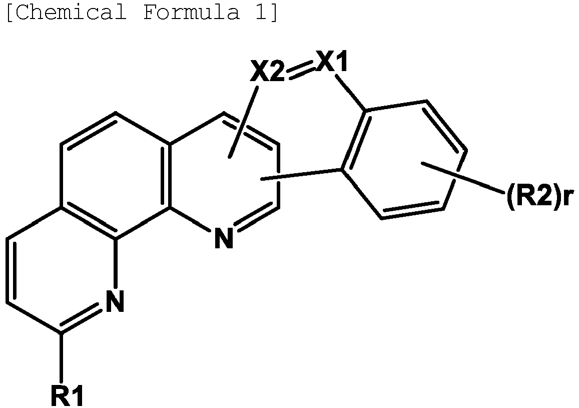

In Chemical Formula 1,
X1 and X2 are each independently N; or CR, and at least one of X1 and X2 is N,
R, R1 and R2 are each independently hydrogen; deuterium; a halogen group; a cyano group; a substituted or unsubstituted silyl group; a substituted or unsubstituted phosphine oxide group; a substituted or unsubstituted C1 to C60 alkyl group; a substituted or unsubstituted C3 to C60 cycloalkyl group; a substituted or unsubstituted C6 to C60 aryl group; or a substituted or unsubstituted C2 to C60 heteroaryl group, and
r is an integer of 1 to 4, and when 2 or greater, R2s are the same as or different from each other.

In addition, one embodiment of the present application provides an organic light emitting device including a first electrode; a second electrode provided opposite to the first electrode; and an organic material layer provided between the first electrode and the second electrode, wherein the organic material layer includes one or more types of the heterocyclic compound of Chemical Formula 1.

### [Advantageous Effects]

A heterocyclic compound described in the present specification can be used as a material of an organic material layer of an organic light emitting device. The heterocyclic compound is capable of acting as a hole injection material, a hole transfer material, a light emitting material, an electron transfer material, an electron injection material, a charge generation material or the like. Particularly, the heterocyclic compound can be used as a material of an electron transfer layer or a charge generation layer of an organic light emitting device.

Chemical Formula 1 has a core structure in which a dicyclic heteroring including N is fused to phenanthroline, and, by strengthening electron-attracting properties in the phenanthroline skeleton, is capable of stabilizing anion radicals generated when electrons are injected, and is capable of enhancing electron transfer ability and charge generation ability through adjusting a band gap and an energy level value in a triplet state (T1) .

In addition, when using the heterocyclic compound of Chemical Formula 1 as a material of an electron transfer layer or a charge generation layer of an organic light emitting device, a driving voltage of the device can be lowered, light efficiency can be enhanced, and lifetime properties of the device can be enhanced.

### [Description of Drawings]

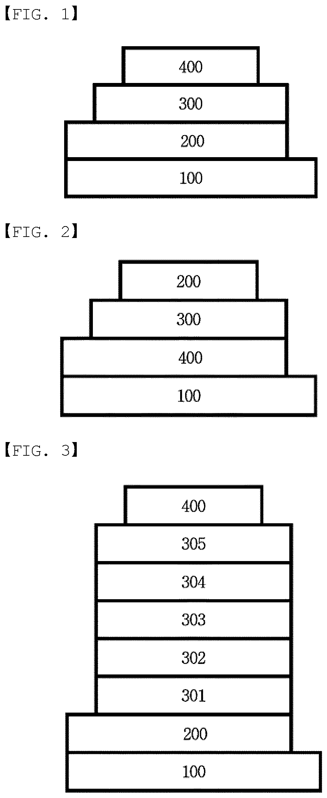

FIG. 1 to FIG. 4 are diagrams each illustrating a lamination structure of an organic light emitting device according to one embodiment of the present specification.
100: Substrate
200: Anode
300: Organic Material Layer
301: Hole Injection Layer
302: Hole Transfer Layer
303: Light Emitting Layer
304: Electron Transfer Layer
305: Electron Injection Layer
400: Cathode

### [Mode for Disclosure]

Hereinafter, the present specification will be described in more detail.

In the present specification, a description of a certain part "including" certain constituents means capable of further including other constituents, and does not exclude other constituents unless particularly stated on the contrary.

In the present specification, a T1 value means an energy level value in a triplet state.

A term "substitution" means a hydrogen atom bonding to a carbon atom of a compound being changed to another substituent, and the position of substitution is not limited as long as it is a position at which the hydrogen atom is substituted, that is, a position at which a substituent is capable of substituting, and when two or more substituents substitute, the two or more substituents may be the same as or different from each other.

In the present specification, "substituted or unsubstituted" means being substituted with one or more substituents selected from the group consisting of deuterium; halogen; a cyano group; a C1 to C60 alkyl group; a C2 to C60 alkenyl group; a C2 to C60 alkynyl group; a C3 to C60 cycloalkyl group; a C2 to C60 heterocycloalkyl group; a C6 to C60 aryl group; a C2 to C60 heteroaryl group; a silyl group; a phosphine oxide group; and an amine group, or being unsubstituted, or being substituted with a substituent linking two or more substituents selected from among the substituents illustrated above, or being unsubstituted.

In the present specification, a "case of a substituent being not indicated in a chemical formula or compound structure" means that a hydrogen atom bonds to a carbon atom. However, since deuterium (²H) is an isotope of hydrogen, some hydrogen atoms may be deuterium.

In one embodiment of the present application, a "case of a substituent being not indicated in a chemical formula or compound structure" may mean that positions that may come as a substituent may all be hydrogen or deuterium. In other words, since deuterium is an isotope of hydrogen, some hydrogen atoms may be deuterium that is an isotope, and herein, a content of the deuterium may be from 0% to 100%.

In one embodiment of the present application, in a "case of a substituent being not indicated in a chemical formula or compound structure", hydrogen and deuterium may be mixed in compounds when deuterium is not explicitly excluded such as a deuterium content being 0%, a hydrogen content being 100% or substituents being all hydrogen.

In one embodiment of the present application, deuterium is one of isotopes of hydrogen, is an element having deuteron formed with one proton and one neutron as a nucleus, and may be expressed as hydrogen-2, and the elemental symbol may also be written as D or ²H.

In one embodiment of the present application, an isotope means an atom with the same atomic number (Z) but with a different mass number (A), and may also be interpreted as an element with the same number of protons but with a different number of neutrons.

In one embodiment of the present application, a meaning of a content T% of a specific substituent may be defined as T2/T1×100=T% when the total number of substituents that a basic compound may have is defined as T1, and the number of specific substituents among these is defined as T2.

In other words, in one example, having a deuterium content of 20% in a phenyl group represented by 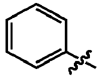 means that the total number of substituents that the phenyl group may have is 5 (T1 in the formula), and the number of deuterium among these is 1 (T2 in the formula). In other words, having a deuterium content of 20% in a phenyl group may be represented by the following structural formulae.

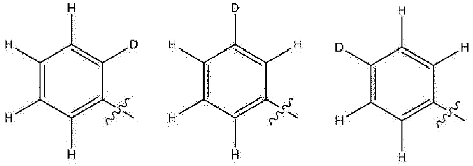

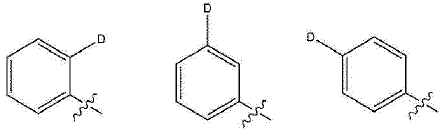

In addition, in one embodiment of the present application, "a phenyl group having a deuterium content of 0%" may mean a phenyl group that does not include a deuterium atom, that is, a phenyl group that has 5 hydrogen atoms.

In the present specification, the halogen may be fluorine, chlorine, bromine or iodine.

In the present specification, the alkyl group includes linear or branched, and may be further substituted with other substituents. The number of carbon atoms of the alkyl group may be from 1 to 60, specifically from 1 to 40 and more specifically from 1 to 20. Specific examples thereof may include a methyl group, an ethyl group, a propyl group, an n-propyl group, an isopropyl group, a butyl group, an n-butyl group, an isobutyl group, a tert-butyl group, a sec-butyl group, a 1-methyl-butyl group, a 1-ethyl-butyl group, a pentyl group, an n-pentyl group, an isopentyl group, a neopentyl group, a tert-pentyl group, a hexyl group, an n-hexyl group, a 1-methylpentyl group, a 2-methylpentyl group, a 4-methyl-2-pentyl group, a 3,3-dimethylbutyl group, a 2-ethylbutyl group, a heptyl group, an n-heptyl group, a 1-methylhexyl group, an octyl group, an n-octyl group, a tert-octyl group, a 1-methylheptyl group, a 2-ethylhexyl group, a 2-propylpentyl group, an n-nonyl group, a 2,2-dimethylheptyl group, a 1-ethyl-propyl group, a 1,1-dimethylpropyl group, an isohexyl group, a 2-methylpentyl group, a 4-methylhexyl group, a 5-methylhexyl group and the like, but are not limited thereto.

In the present specification, the alkenyl group includes linear or branched, and may be further substituted with other substituents. The number of carbon atoms of the alkenyl group may be from 2 to 60, specifically from 2 to 40 and more specifically from 2 to 20. Specific examples thereof may include a vinyl group, a 1-propenyl group, an isopropenyl group, a 1-butenyl group, a 2-butenyl group, a 3-butenyl group, a 1-pentenyl group, a 2-pentenyl group, a 3-pentenyl group, a 3-methyl-1-butenyl group, a 1,3-butadienyl group, an allyl group, a 1-phenylvinyl-1-yl group, a 2-phenylvinyl-1-yl group, a 2,2-diphenylvinyl-1-yl group, a 2-phenyl-2-(naphthyl-1-yl)vinyl-1-yl group, a 2,2-bis(diphenyl-1-yl)vinyl-1-yl group, a stilbenyl group, a styrenyl group and the like, but are not limited thereto.

In the present specification, the alkynyl group includes linear or branched, and may be further substituted with other substituents. The number of carbon atoms of the alkynyl group may be from 2 to 60, specifically from 2 to 40 and more specifically from 2 to 20.

In the present specification, the cycloalkyl group includes monocyclic or polycyclic having 3 to 60 carbon atoms, and may be further substituted with other substituents. Herein, the polycyclic means a group in which the cycloalkyl group is directly linked to or fused with other cyclic groups. Herein, the other cyclic groups may be a cycloalkyl group, but may also be different types of cyclic groups such as a heterocycloalkyl group, an aryl group and a heteroaryl group. The number of carbon groups of the cycloalkyl group may be from 3 to 60, specifically from 3 to 40 and more specifically from 5 to 20. Specific examples thereof may include a cyclopropyl group, a cyclobutyl group, a cyclopentyl group, a 3-methylcyclopentyl group, a 2,3-dimethylcyclopentyl group, a cyclohexyl group, a 3-methylcyclohexyl group, a 4-methylcyclohexyl group, a 2,3-dimethylcyclohexyl group, a 3,4,5-trimethylcyclohexyl group, a 4-tert-butylcyclohexyl group, a cycloheptyl group, a cyclooctyl group and the like, but are not limited thereto.

In the present specification, the heterocycloalkyl group includes O, S, Se, N or Si as a heteroatom, includes monocyclic or polycyclic having 2 to 60 carbon atoms, and may be further substituted with other substituents. Herein, the polycyclic means a group in which the heterocycloalkyl group is directly linked to or fused with other cyclic groups. Herein, the other cyclic groups may be a heterocycloalkyl group, but may also be different types of cyclic groups such as a cycloalkyl group, an aryl group and a heteroaryl group. The number of carbon atoms of the heterocycloalkyl group may be from 2 to 60, specifically from 2 to 40 and more specifically from 3 to 20.

In the present specification, the aryl group includes monocyclic or polycyclic having 6 to 60 carbon atoms, and may be further substituted with other substituents. Herein, the polycyclic means a group in which the aryl group is directly linked to or fused with other cyclic groups. Herein, the other cyclic groups may be an aryl group, but may also be different types of cyclic groups such as a cycloalkyl group, a heterocycloalkyl group and a heteroaryl group. The aryl group includes a spiro group. The number of carbon atoms of the aryl group may be from 6 to 60, specifically from 6 to 40 and more specifically from 6 to 25. Specific examples of the aryl group may include a phenyl group, a biphenyl group, a terphenyl group, a naphthyl group, an anthryl group, a chrysenyl group, a phenanthrenyl group, a perylenyl group, a fluoranthenyl group, a triphenylenyl group, a phenalenyl group, a pyrenyl group, a tetracenyl group, a pentacenyl group, a fluorenyl group, an indenyl group, an acenaphthylenyl group, a benzofluorenyl group, a spirobifluorenyl group, a 2,3-dihydro-1H-indenyl group, a fused ring group thereof, and the like, but are not limited thereto.

In the present specification, the terphenyl group may be selected from among the following structures.

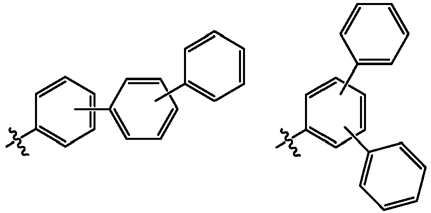

In the present specification, the fluorenyl group may be substituted, and adjacent substituents may bond to each other to form a ring. When the fluorenyl group is substituted, 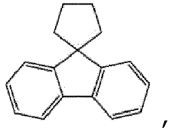 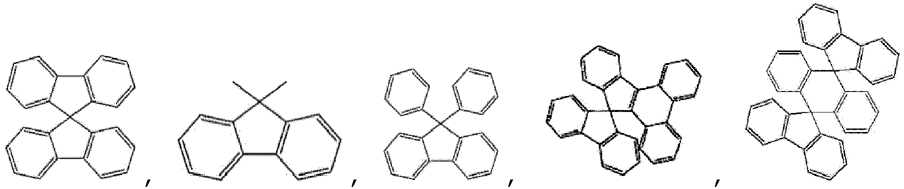 and the like may be included, however, the structure is not limited thereto.

In the present specification, the heteroaryl group includes O, S, SO₂, Se, N or Si as a heteroatom, includes monocyclic or polycyclic, and may be further substituted with other substituents. Herein, the polycyclic means a group in which the heteroaryl group is directly linked to or fused with other cyclic groups. Herein, the other cyclic groups may be a heteroaryl group, but may also be different types of cyclic groups such as a cycloalkyl group, a heterocycloalkyl group and an aryl group. The number of carbon atoms of the heteroaryl group may be from 2 to 60, specifically from 2 to 40 and more specifically from 3 to 25. Specific examples of the heteroaryl group may include a pyridyl group, a pyrrolyl group, a pyrimidyl group, a pyridazinyl group, a furanyl group, a thiophene group, an imidazolyl group, a pyrazolyl group, an oxazolyl group, an isoxazolyl group, a thiazolyl group, an isothiazolyl group, a triazolyl group, a furazanyl group, an oxadiazolyl group, a thiadiazolyl group, a dithiazolyl group, a tetrazolyl group, a pyranyl group, a thiopyranyl group, a diazinyl group, an oxazinyl group, a thiazinyl group, a dioxynyl group, a triazinyl group, a tetrazinyl group, a quinolyl group, an isoquinolyl group, a quinazolinyl group, an isoquinazolinyl group, a qninozolinyl group, a naphthyridyl group, an acridinyl group, a phenanthridinyl group, an imidazopyridinyl group, a diazanaphthalenyl group, a triazaindene group, an indolyl group, an indolizinyl group, a benzothiazolyl group, a benzoxazolyl group, a benzimidazolyl group, a benzothiophene group, a benzofuran group, a dibenzothiophene group, a dibenzofuran group, a carbazolyl group, a benzocarbazolyl group, a dibenzocarbazolyl group, a phenazinyl group, a dibenzosilole group, spirobi(dibenzosilole), a dihydrophenazinyl group, a phenoxazinyl group, a phenanthridyl group, an imidazopyridinyl group, a thienyl group, an indolo[2,3-a]carbazolyl group, an indolo[2,3-b]carbazolyl group, an indolinyl group, a 10,11-dihydro-dibenzo[b,f]azepine group, a 9,10-dihydroacridinyl group, a phenanthrazinyl group, a phenothiathiazinyl group, a phthalazinyl group, a naphthylidinyl group, a phenanthrolinyl group, a benzo[c][1,2,5]thiadiazolyl group, 5,10-dihydrobenzo[b,e] [1,4]azasilinyl, a pyrazolo[1,5-c]quinazolinyl group, a pyrido[1,2-b]indazolyl group, a pyrido[1,2-a]imidazo[1,2-e]indolinyl group, a benzofuro[2,3-d]pyrimidyl group; a benzothieno[2,3-d]pyrimidyl group; a benzofuro[2,3-a]carbazolyl group, a benzothieno[2,3-a]carbazolyl group, a 1,3-dihydroindolo[2,3-a]carbazolyl group, a benzofuro[3,2-a]carbazolyl group, a benzothieno[3,2-a]carbazolyl group, a 1,3-dihydroindolo[3,2-a]carbazolyl group, a benzofuro[2,3-b]carbazolyl group, a benzothieno[2,3-b]carbazolyl group, a 1,3-dihydroindolo[2,3-b]carbazolyl group, a benzofuro[3,2-b]carbazolyl group, a benzothieno[3,2-b]carbazolyl group, a 1,3-dihydroindolo[3,2-b]carbazolyl group, a benzofuro[2,3-c]carbazolyl group, a benzothieno[2,3-c]carbazolyl group, a 1,3-dihydroindolo[2,3-c]carbazolyl group, a benzofuro[3,2-c]carbazolyl group, a benzothieno[3,2-c]carbazolyl group, a 1,3-dihydroindolo[3,2-c]carbazolyl group, a 1,3-dihydroindeno[2,1-b]carbazolyl group, a 5,11-dihydroindeno[1,2-b]carbazolyl group, a 5,12-dihydroindeno[1,2-c]carbazolyl group, a 5,8-dihydroindeno[2,1-c]carbazolyl group, a 7,12-dihydroindeno[1,2-a]carbazolyl group, a 11,12-dihydroindeno[2,1-a]carbazolyl group and the like, but are not limited thereto.

In the present specification, the silyl group is a substituent including Si, having the Si atom directly linked as a radical, and is represented by -SiR₁₀₁R₁₀₂R₁₀₃. R₁₀₁ to R₁₀₃ are the same as or different from each other, and may be each independently a substituent formed with at least one of hydrogen; deuterium; a halogen group; an alkyl group; an alkenyl group; an alkoxy group; a cycloalkyl group; an aryl group; and a heteroaryl group. Specific examples of the silyl group may include a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group and the like, but are not limited thereto.

In the present specification, the phosphine oxide group is represented by -P (=O) R₁₀₄R₁₀₅, and R₁₀₄ and R₁₀₅ are the same as or different from each other and may be each independently a substituent formed with at least one of hydrogen; deuterium; a halogen group; an alkyl group; an alkenyl group; an alkoxy group; a cycloalkyl group; an aryl group; and a heteroaryl group. Specifically, the phosphine oxide group may be substituted with an aryl group, and as the aryl group, the examples described above may be applied. Examples of the phosphine oxide group may include a dimethylphosphine oxide group, a diphenylphosphine oxide group, a dinaphthylphosphine oxide group and the like, but are not limited thereto.

In the present specification, the amine group is represented by -NR₁₀₆R₁₀₇, and R₁₀₆ and R₁₀₇ are the same as or different from each other and may be each independently a substituent formed with at least one of hydrogen; deuterium; a halogen group; an alkyl group; an alkenyl group; an alkoxy group; a cycloalkyl group; an aryl group; and a heteroaryl group. Specifically, the amine group may be selected from the group consisting of -NH₂; a monoalkylamine group; a monoarylamine group; a monoheteroarylamine group; a dialkylamine group; a diarylamine group; a diheteroarylamine group; an alkylarylamine group; an alkylheteroarylamine group; and an arylheteroarylamine group, and although not particularly limited thereto, the number of carbon atoms is preferably from 1 to 30. Specific examples of the amine group may include a methylamine group, a dimethylamine group, an ethylamine group, a diethylamine group, a phenylamine group, a naphthylamine group, a biphenylamine group, a dibiphenylamine group, an anthracenylamine group, a 9-methyl-anthracenylamine group, a diphenylamine group, a phenylnaphthylamine group, a ditolylamine group, a phenyltolylamine group, a triphenylamine group, a biphenylnaphthylamine group, a phenylbiphenylamine group, a biphenylfluorenylamine group, a phenyltriphenylenylamine group, a biphenyltriphenylenylamine group and the like, but are not limited thereto.

In the present specification, the examples of the aryl group described above may be applied to the arylene group except that the arylene group is a divalent group.

In the present specification, the examples of the heteroaryl group described above may be applied to the heteroarylene group except that the heteroarylene group is a divalent group.

In the present specification, an "adjacent group" may mean a substituent substituting an atom directly linked to an atom substituted by the corresponding substituent, a substituent sterically most closely positioned to the corresponding substituent, or another substituent substituting an atom substituted by the corresponding substituent. For example, two substituents substituting ortho positions in a benzene ring, and two substituents substituting the same carbon in an aliphatic ring may be interpreted as an "adjacent group" to each other.

As the aliphatic hydrocarbon ring, the aromatic hydrocarbon ring, the aliphatic heteroring or the aromatic heteroring that adjacent groups may form, the structures illustrated as the cycloalkyl group, the aryl group, the heterocycloalkyl group and the heteroaryl group described above may be used respectively except for those that are not a monovalent group.

One embodiment of the present specification provides a heterocyclic compound represented by Chemical Formula 1.

In one embodiment of the present specification, X1 and X2 are each independently N; or CR, and at least one of X1 and X2 is N.

In one embodiment of the present specification, X1 is N, and X2 is CR.

In one embodiment of the present specification, X1 is CR, and X2 is N.

In one embodiment of the present specification, R and R1 are each independently hydrogen; deuterium; a halogen group; a cyano group; a substituted or unsubstituted silyl group; a substituted or unsubstituted phosphine oxide group; a substituted or unsubstituted C1 to C60 alkyl group; a substituted or unsubstituted C3 to C60 cycloalkyl group; a substituted or unsubstituted C6 to C60 aryl group; or a substituted or unsubstituted C2 to C60 heteroaryl group.

In one embodiment of the present specification, R and R1 are each independently hydrogen; deuterium; a halogen group; a cyano group; a substituted or unsubstituted silyl group; a substituted or unsubstituted phosphine oxide group; a substituted or unsubstituted C6 to C60 aryl group; or a substituted or unsubstituted C2 to C60 heteroaryl group.

In one embodiment of the present specification, R and R1 are each independently hydrogen; deuterium; a substituted or unsubstituted phosphine oxide group; a substituted or unsubstituted C6 to C60 aryl group; or a substituted or unsubstituted C2 to C60 heteroaryl group.

In one embodiment of the present specification, R and R1 are each independently hydrogen; deuterium; a substituted or unsubstituted phosphine oxide group; a substituted or unsubstituted C6 to C30 aryl group; or a substituted or unsubstituted C2 to C30 heteroaryl group.

In one embodiment of the present specification, R is hydrogen; deuterium; a substituted or unsubstituted phosphine oxide group; a substituted or unsubstituted C6 to C60 aryl group; or a substituted or unsubstituted C2 to C60 heteroaryl group.

In one embodiment of the present specification, R is hydrogen; deuterium; a substituted or unsubstituted phosphine oxide group; a substituted or unsubstituted C6 to C30 aryl group; or a substituted or unsubstituted C2 to C30 heteroaryl group.

In one embodiment of the present specification, R is a substituted or unsubstituted phosphine oxide group; a substituted or unsubstituted C6 to C30 aryl group; or a substituted or unsubstituted C2 to C30 heteroaryl group.

In one embodiment of the present specification, R1 is hydrogen; deuterium; a substituted or unsubstituted C6 to C60 aryl group; or a substituted or unsubstituted C2 to C60 heteroaryl group.

In one embodiment of the present specification, R1 is hydrogen; deuterium; a substituted or unsubstituted C6 to C30 aryl group; or a substituted or unsubstituted C2 to C30 heteroaryl group.

In one embodiment of the present specification, R1 is hydrogen; deuterium; a substituted or unsubstituted phenyl group; a substituted or unsubstituted naphthyl group; or a substituted or unsubstituted phenanthroline group.

In one embodiment of the present specification, R1 is a substituted or unsubstituted C6 to C30 aryl group; or a substituted or unsubstituted C2 to C30 heteroaryl group.

In one embodiment of the present specification, R1 is a substituted or unsubstituted phenyl group; a substituted or unsubstituted naphthyl group; or a substituted or unsubstituted phenanthroline group.

In one embodiment of the present specification, R2 is hydrogen; deuterium; a substituted or unsubstituted C6 to C60 aryl group; or a substituted or unsubstituted C2 to C60 heteroaryl group.

In one embodiment of the present specification, R2 is hydrogen; deuterium; a substituted or unsubstituted C6 to C30 aryl group; or a substituted or unsubstituted C2 to C30 heteroaryl group.

In one embodiment of the present specification, R2 is hydrogen; deuterium; a substituted or unsubstituted C6 to C20 aryl group; or a substituted or unsubstituted C2 to C20 heteroaryl group.

In one embodiment of the present specification, R2 is hydrogen; deuterium; a substituted or unsubstituted phenyl group; a substituted or unsubstituted naphthyl group; a substituted or unsubstituted pyridine group; a substituted or unsubstituted pyrimidine group; a substituted or unsubstituted triazine group; or a substituted or unsubstituted phenanthroline group.

In one embodiment of the present specification, R2 is hydrogen; deuterium; a C6 to C20 aryl group unsubstituted or substituted with a heteroaryl group; or a C2 to C20 heteroaryl group unsubstituted or substituted with an aryl group or a heteroaryl group.

In one embodiment of the present specification, R1 and R2 are hydrogen; or deuterium.

In one embodiment of the present specification, R1 is a substituted or unsubstituted C6 to C30 aryl group; or a substituted or unsubstituted C2 to C30 heteroaryl group, and R2 is hydrogen; or deuterium.

In one embodiment of the present specification, R1 is a substituted or unsubstituted phenyl group; a substituted or unsubstituted naphthyl group; or a substituted or unsubstituted phenanthroline group, and R2 is hydrogen; or deuterium.

In one embodiment of the present specification, R1 is hydrogen, and R2 is a substituted or unsubstituted C6 to C30 aryl group; or a substituted or unsubstituted C2 to C30 heteroaryl group.

In one embodiment of the present specification, R1 is hydrogen, and R2 is a substituted or unsubstituted phenyl group; a substituted or unsubstituted naphthyl group; or a substituted or unsubstituted phenanthroline group.

In one embodiment of the present specification, R1 and R2 are each independently a substituted or unsubstituted C6 to C30 aryl group; or a substituted or unsubstituted C2 to C30 heteroaryl group.

In one embodiment of the present specification, Chemical Formula 1 may be represented by the following Chemical Formula 1-1 or Chemical Formula 1-2.

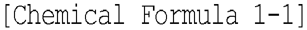

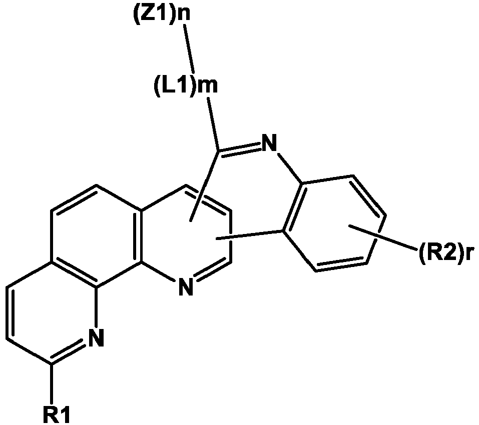

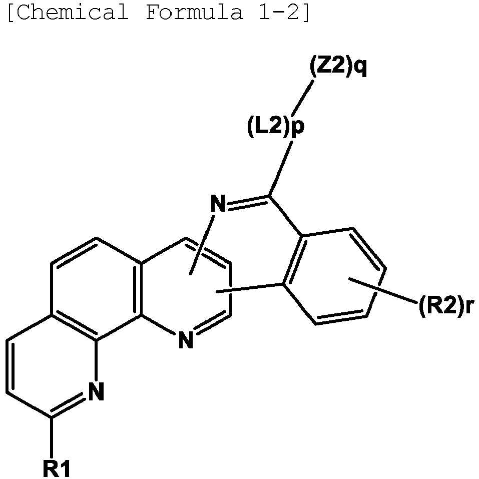

In Chemical Formulae 1-1 and 1-2,
R1, R2 and r have the same definitions as in Chemical Formula 1,
L1 and L2 are each independently a direct bond; a substituted or unsubstituted C6 to C60 arylene group; or a substituted or unsubstituted C2 to C60 heteroarylene group,
Z1 and Z2 are each independently a halogen group; a cyano group; a substituted or unsubstituted silyl group; a substituted or unsubstituted phosphine oxide group; a substituted or unsubstituted C1 to C60 alkyl group; a substituted or unsubstituted C3 to C60 cycloalkyl group; a substituted or unsubstituted C6 to C60 aryl group; or a substituted or unsubstituted C2 to C60 heteroaryl group,
m is an integer of 1 to 5, and when 2 or greater, L1s are the same as or different from each other,
n is an integer of 1 to 5, and when 2 or greater, Z1s are the same as or different from each other,
p is an integer of 1 to 5, and when 2 or greater, L2s are the same as or different from each other, and
q is an integer of 1 to 5, and when 2 or greater, Z2s are the same as or different from each other.

In one embodiment of the present specification, Chemical Formula 1 may be represented by any one of the following Chemical Formulae 2-1 to 2-4.

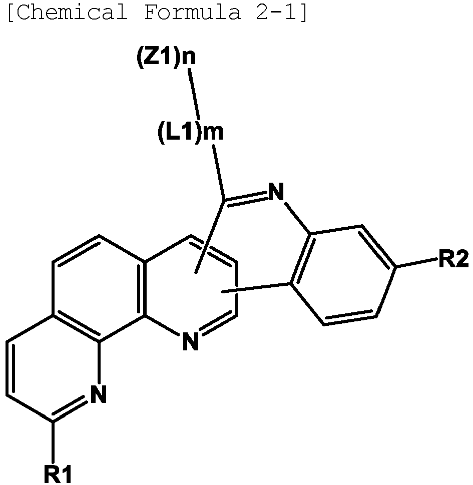

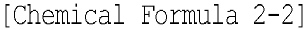

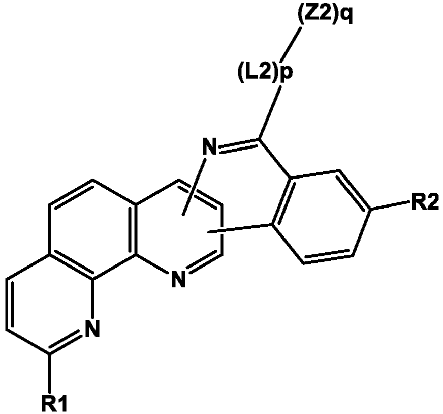

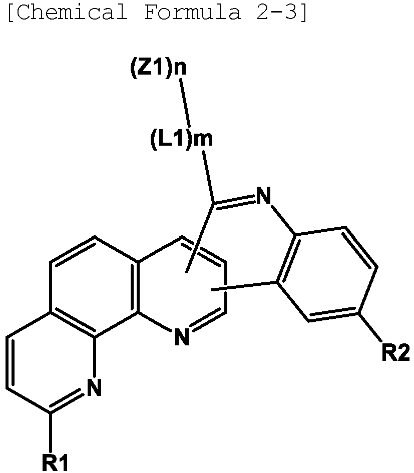

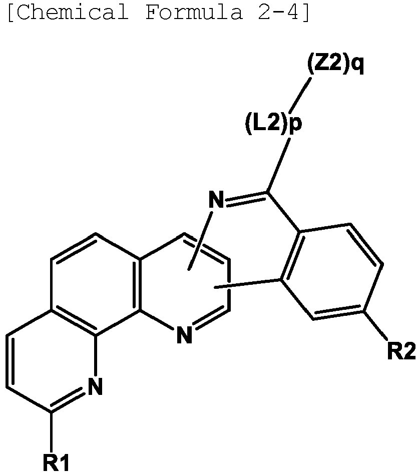

In Chemical Formulae 2-1 to 2-4,
R1 and R2 have the same definitions as in Chemical Formula 1,
L1 and L2 are each independently a direct bond; a substituted or unsubstituted C6 to C60 arylene group; or a substituted or unsubstituted C2 to C60 heteroarylene group,
Z1 and Z2 are each independently a halogen group; a cyano group; a substituted or unsubstituted silyl group; a substituted or unsubstituted phosphine oxide group; a substituted or unsubstituted C1 to C60 alkyl group; a substituted or unsubstituted C3 to C60 cycloalkyl group; a substituted or unsubstituted C6 to C60 aryl group; or a substituted or unsubstituted C2 to C60 heteroaryl group,
m is an integer of 1 to 5, and when 2 or greater, L1s are the same as or different from each other,
n is an integer of 1 to 5, and when 2 or greater, Z1s are the same as or different from each other,
p is an integer of 1 to 5, and when 2 or greater, L2s are the same as or different from each other, and
q is an integer of 1 to 5, and when 2 or greater, Z2s are the same as or different from each other.

In one embodiment of the present specification, L1 and L2 are each independently a direct bond; a substituted or unsubstituted C6 to C60 arylene group; or a substituted or unsubstituted C2 to C60 heteroarylene group.

In one embodiment of the present specification, L1 and L2 are each independently a direct bond; a substituted or unsubstituted C6 to C30 arylene group; or a substituted or unsubstituted C2 to C30 heteroarylene group.

In one embodiment of the present specification, L1 and L2 are each independently a direct bond; a substituted or unsubstituted phenylene group; a substituted or unsubstituted anthracenylene group; a substituted or unsubstituted divalent pyridine group; a substituted or unsubstituted divalent pyrimidine group; a substituted or unsubstituted divalent triazine group; or a substituted or unsubstituted divalent phenanthroline group.

In one embodiment of the present specification, Z1 and Z2 are each independently a substituted or unsubstituted phosphine oxide group; a substituted or unsubstituted C6 to C60 aryl group; or a substituted or unsubstituted C2 to C60 heteroaryl group.

In one embodiment of the present specification, Z1 and Z2 are each independently a substituted or unsubstituted phosphine oxide group; a substituted or unsubstituted C6 to C30 aryl group; or a substituted or unsubstituted C2 to C30 heteroaryl group.

In one embodiment of the present specification, Z1 and Z2 are each independently a substituted or unsubstituted phosphine oxide group; a substituted or unsubstituted phenyl group; a substituted or unsubstituted biphenyl group; a substituted or unsubstituted terphenyl group; a substituted or unsubstituted naphthyl group; a substituted or unsubstituted anthracene group; a substituted or unsubstituted phenanthrene group; a substituted or unsubstituted triphenylene group; a substituted or unsubstituted fluorenyl group; a substituted or unsubstituted pyridine group; a substituted or unsubstituted pyrimidine group; a substituted or unsubstituted triazine group; or a substituted or unsubstituted phenanthroline group.

In one embodiment of the present specification, Z1 and Z2 are each independently a phosphine oxide group unsubstituted or substituted with an aryl group; a phenyl group unsubstituted or substituted with an aryl group or a heteroaryl group; a biphenyl group; a terphenyl group; a naphthyl group; an anthracene group unsubstituted or substituted with an aryl group; a phenanthrene group; a substituted or unsubstituted triphenylene group; 9,9'-spirobi[fluorene]; a pyridine group unsubstituted or substituted with an aryl group or a heteroaryl group; a pyrimidine group unsubstituted or substituted with an aryl group; a triazine group unsubstituted or substituted with an aryl group; or a phenanthroline group unsubstituted or substituted with an aryl group.

In one embodiment of the present specification, Z1 and Z2 may be each independently represented by any one of the following Chemical Formulae A to C.

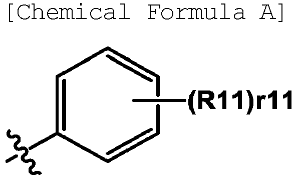

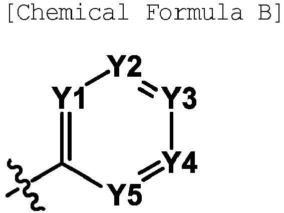

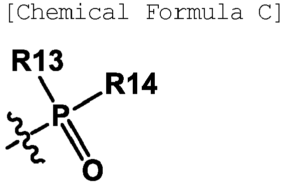

In Chemical Formulae A to C,
R11 is hydrogen; deuterium; a substituted or unsubstituted silyl group; a substituted or unsubstituted phosphine oxide group; a substituted or unsubstituted C1 to C60 alkyl group; a substituted or unsubstituted C3 to C60 cycloalkyl group; a substituted or unsubstituted C6 to C60 aryl group; or a substituted or unsubstituted C2 to C60 heteroaryl group, or adjacent groups may bond to each other to form a substituted or unsubstituted hydrocarbon ring,
r11 is an integer of 1 to 5, and when 2 or greater, R11s are the same as or different from each other,
Y1 to Y5 are each independently N or CR12, at least one thereof is N, and adjacent groups may bond to each other to form a heteroring, and
R12 to R14 are each independently hydrogen; deuterium; a substituted or unsubstituted C6 to C60 aryl group; or a substituted or unsubstituted C2 to C60 heteroaryl group.

In one embodiment of the present specification, R11 is hydrogen; deuterium; a substituted or unsubstituted phosphine oxide group; a substituted or unsubstituted C6 to C60 aryl group; or a substituted or unsubstituted C2 to C60 heteroaryl group, or adjacent groups may bond to each other to form a substituted or unsubstituted hydrocarbon ring.

In one embodiment of the present specification, R11 is hydrogen; deuterium; a substituted or unsubstituted phosphine oxide group; a substituted or unsubstituted C6 to C30 aryl group; or a substituted or unsubstituted C2 to C30 heteroaryl group, or adjacent groups may bond to each other to form a substituted or unsubstituted hydrocarbon ring.

In one embodiment of the present specification, adjacent groups among R11s may bond to each other to form a substituted or unsubstituted C6 to C30 hydrocarbon ring.

In one embodiment of the present specification, adjacent groups among R11s may bond to each other to form a 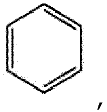 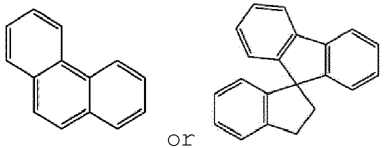 ring, and the ring may be further substituted with an aryl group or a heteroaryl group.

In one embodiment of the present specification, Y1 to Y5 are each independently N or CR12, at least one thereof is N, and adjacent groups may bond to each other to form a heteroring.

In one embodiment of the present specification, Y1 to Y5 are each independently N or CR12, and at least two thereof may be N.

In one embodiment of the present specification, Y1 to Y5 are each independently N or CR12, and at least three thereof may be N.

In one embodiment of the present specification, Y1 to Y5 are each independently N or CR12, at least one thereof is N, and adjacent groups may bond to each other to form a C2 to C30 heteroring.

In one embodiment of the present specification, Y1 to Y5 are each independently N or CR12, at least one thereof is N, adjacent groups may bond to each other to form a 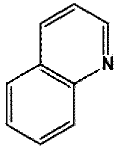 ring, and the ring may be further substituted with an aryl group or a heteroaryl group.

In one embodiment of the present specification, R12 to R14 are each independently hydrogen; deuterium; a substituted or unsubstituted C6 to C60 aryl group; or a substituted or unsubstituted C2 to C60 heteroaryl group.

In one embodiment of the present specification, R12 to R14 are each independently hydrogen; deuterium; a substituted or unsubstituted C6 to C30 aryl group; or a substituted or unsubstituted C2 to C30 heteroaryl group.

In one embodiment of the present specification, Chemical Formula 1 may be represented by any one of the following Chemical Formula 3-1 to Chemical Formula 3-4.

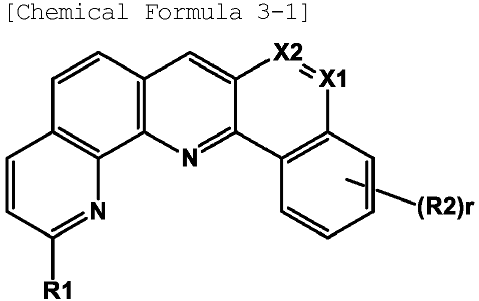

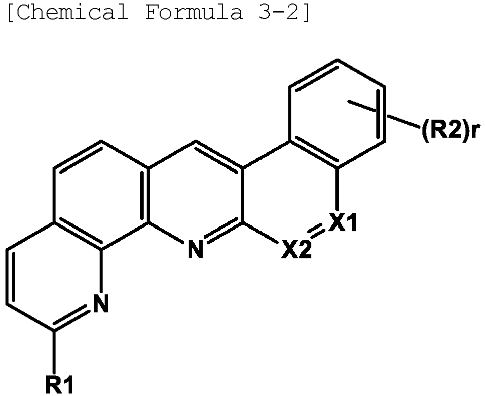

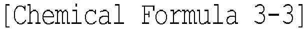

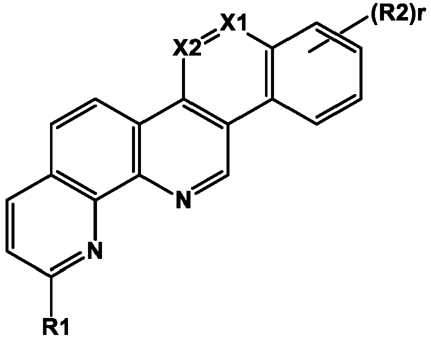

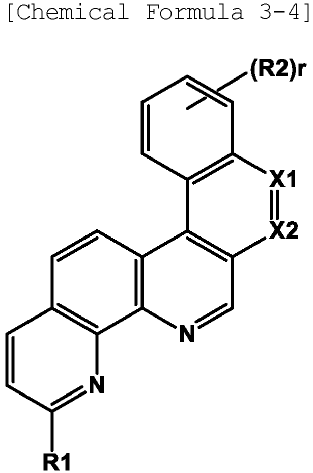

In Chemical Formulae 3-1 to 3-4, each substituent has the same definition as in Chemical Formula 1.

In one embodiment of the present specification, Chemical Formula 1 may be represented by any one of the following Chemical Formula 4-1 to Chemical Formula 4-4.

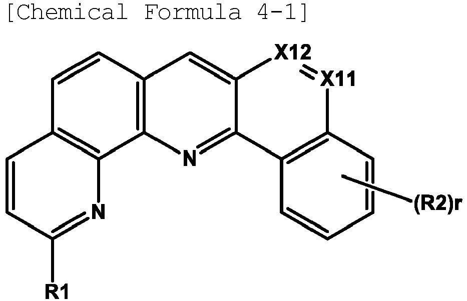

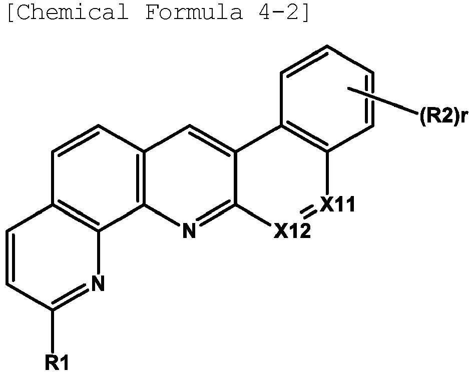

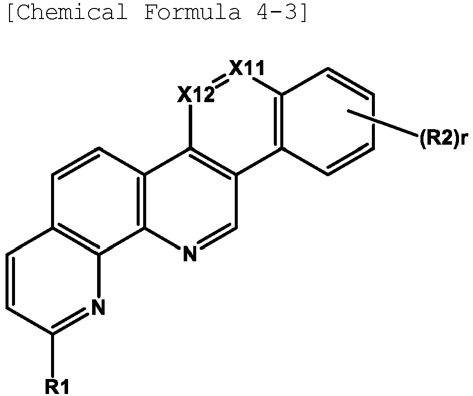

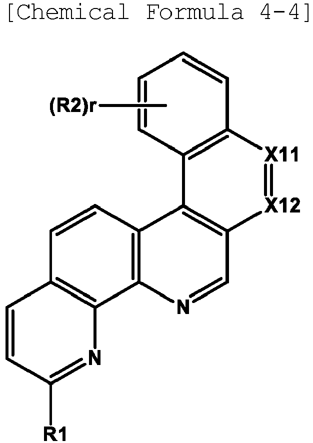

In Chemical Formulae 4-1 to 4-4,
any one of X11 and X12 is N, and the other one is CR, and
R, R1, R2 and r each have the same definition as in Chemical Formula 1.

In one embodiment of the present specification, X11 is N, and X12 is CR.

In one embodiment of the present specification, X11 is CR, and X12 is N.

In one embodiment of the present specification, Chemical Formula 1 may be represented by any one of the following compounds, but is not limited thereto.

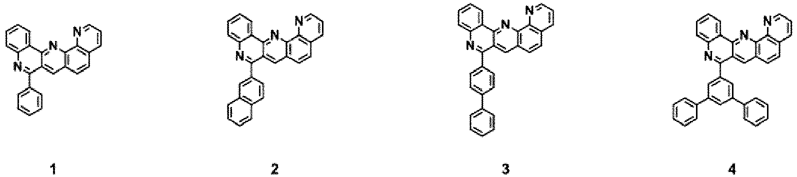

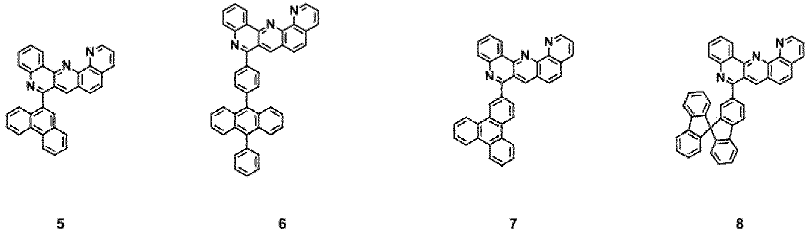

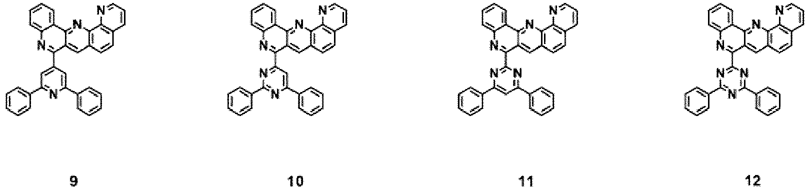

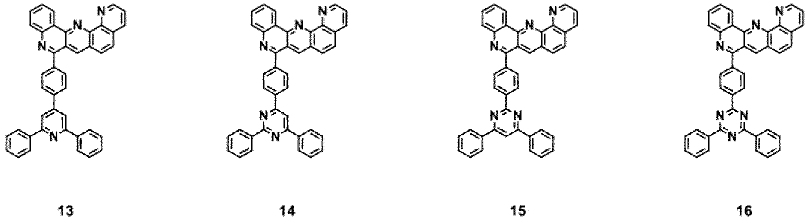

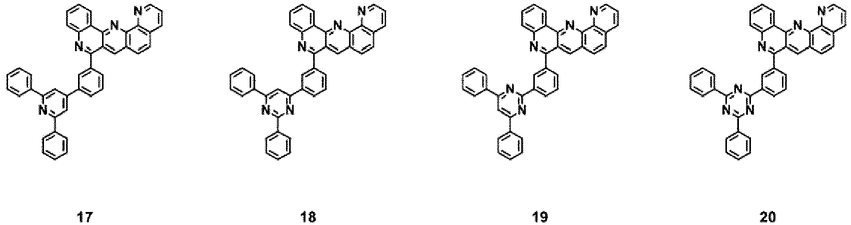

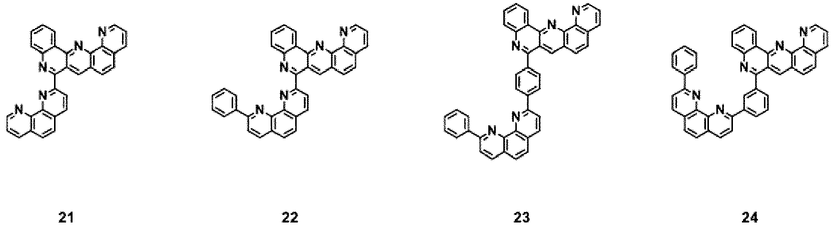

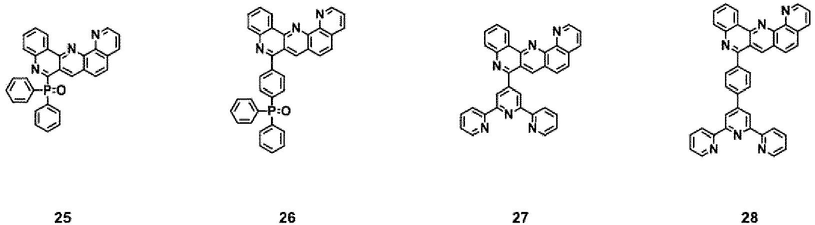

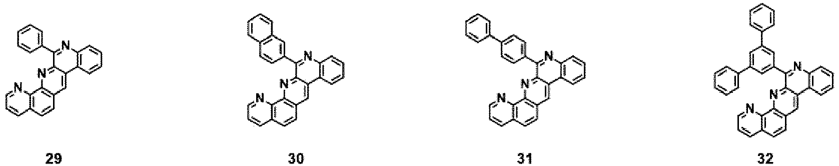

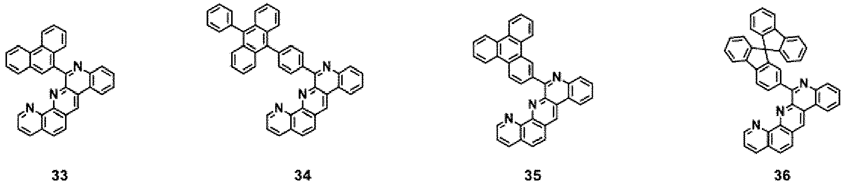

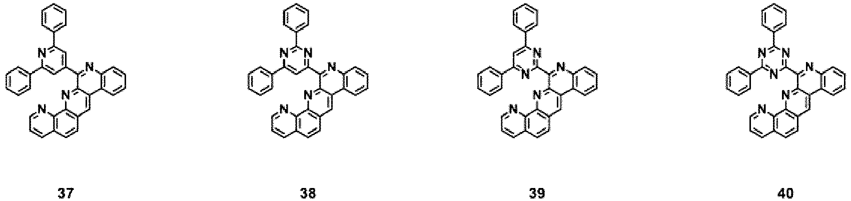

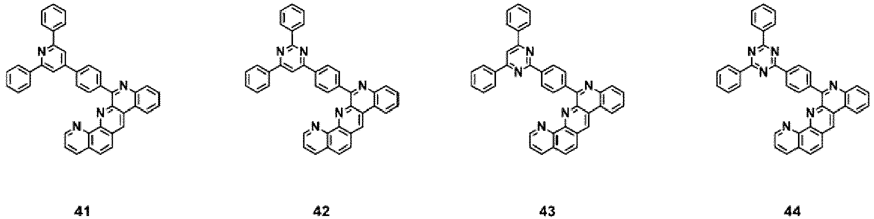

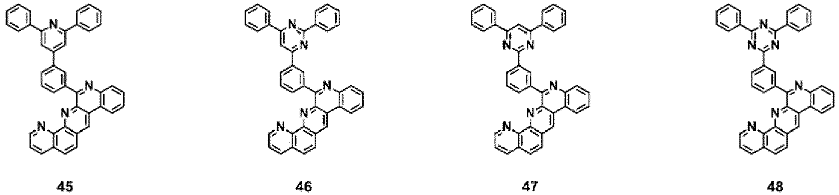

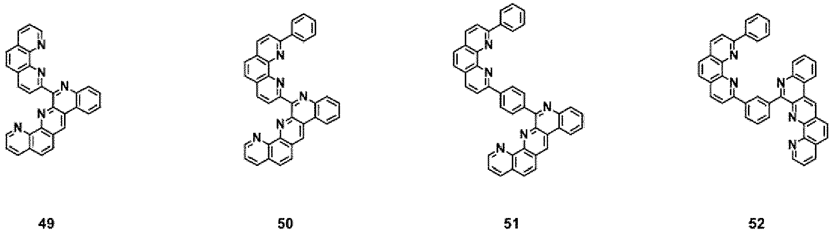

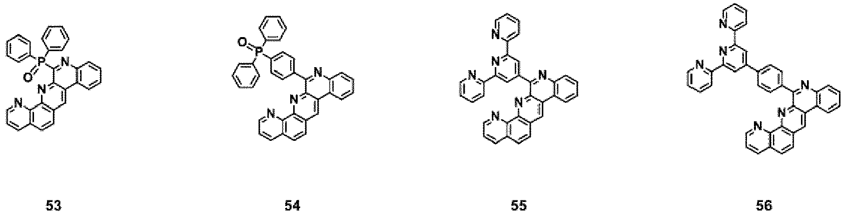

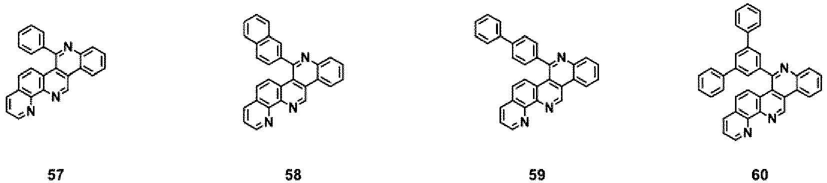

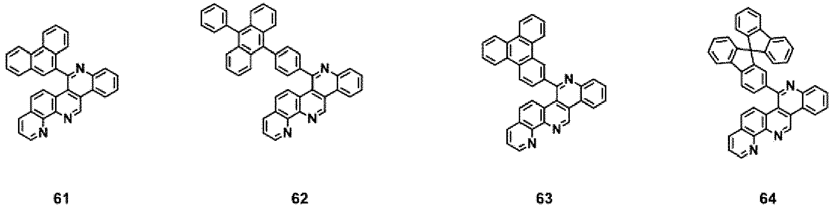

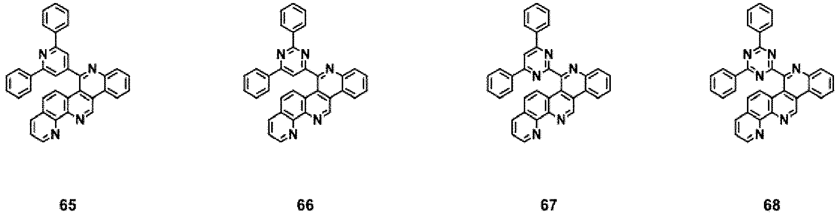

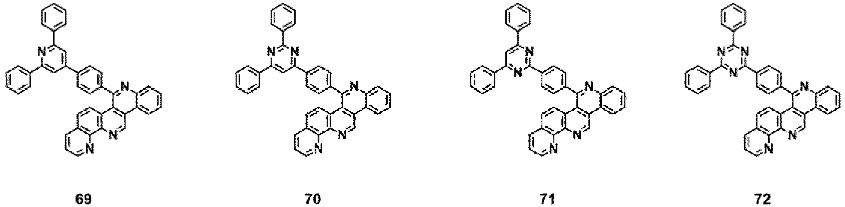

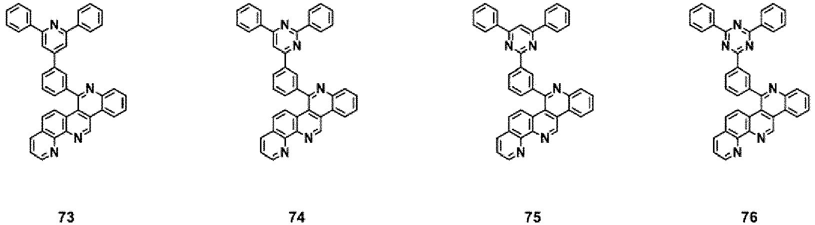

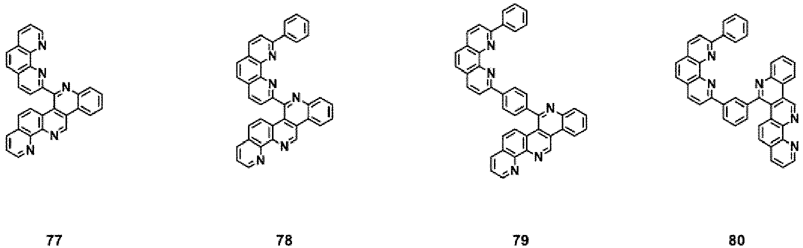

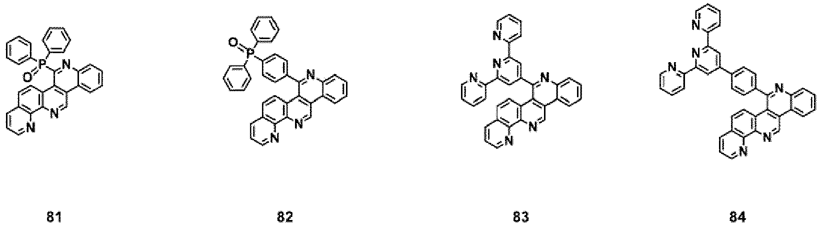

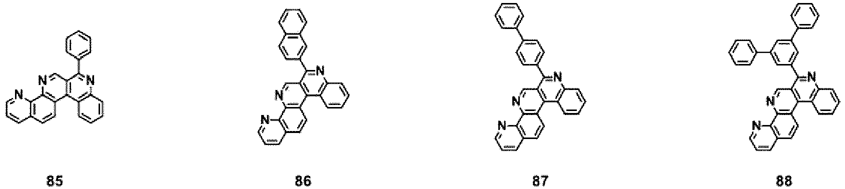

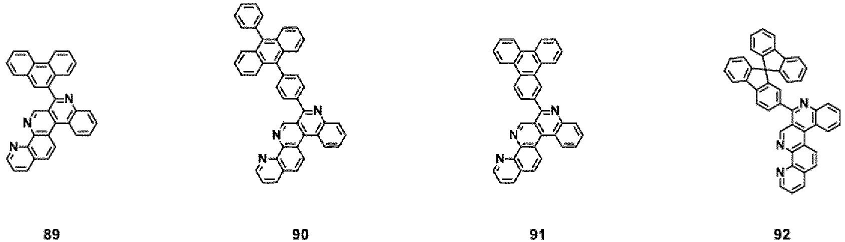

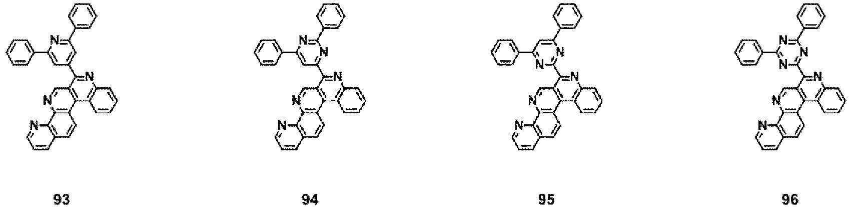

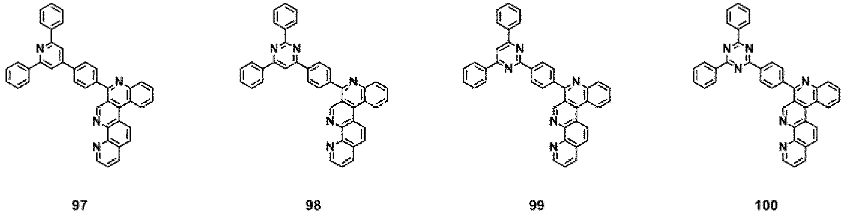

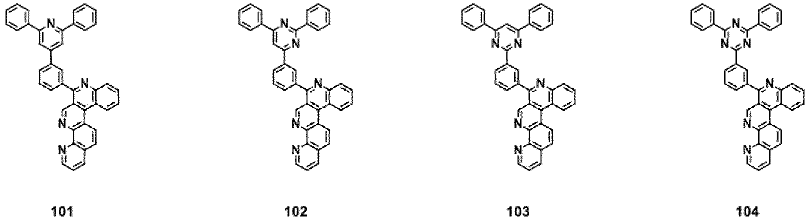

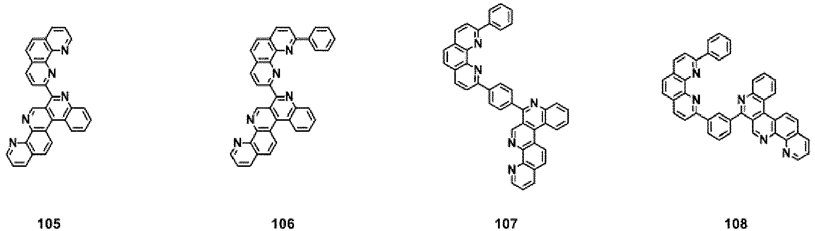

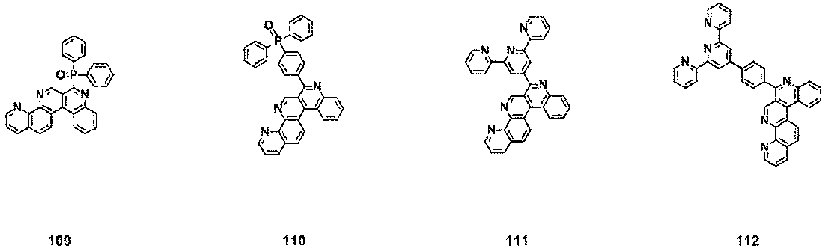

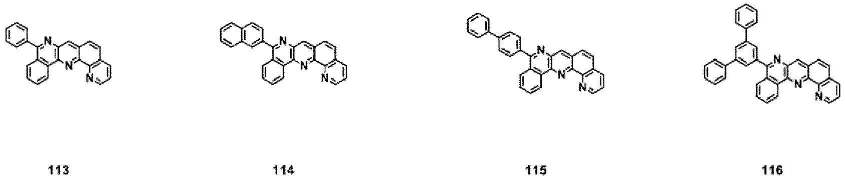

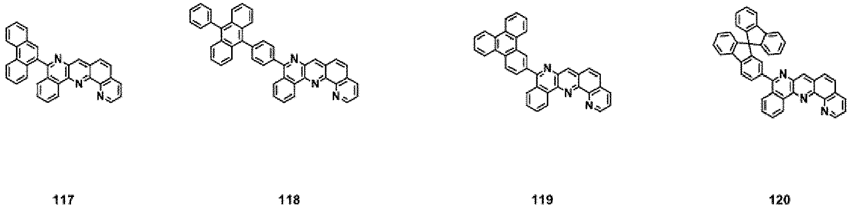

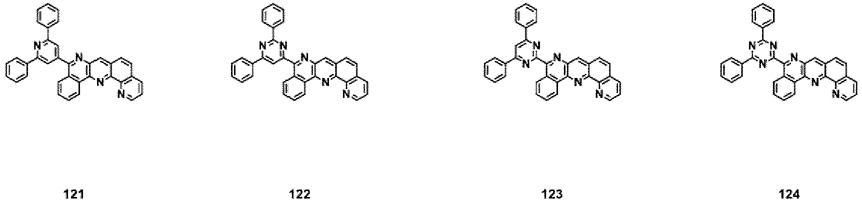

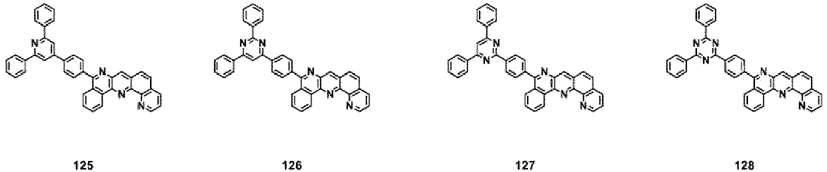

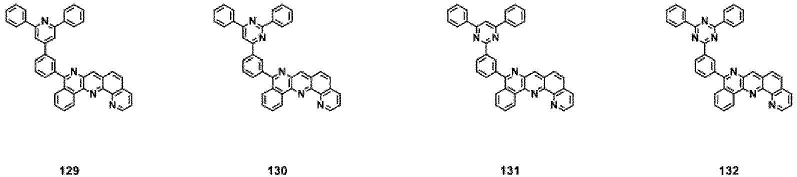

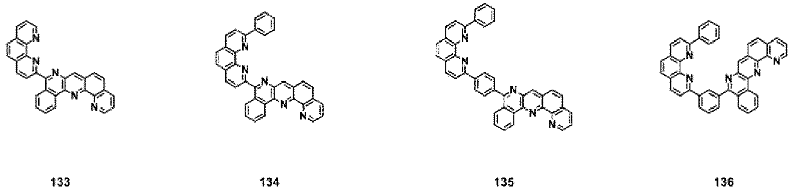

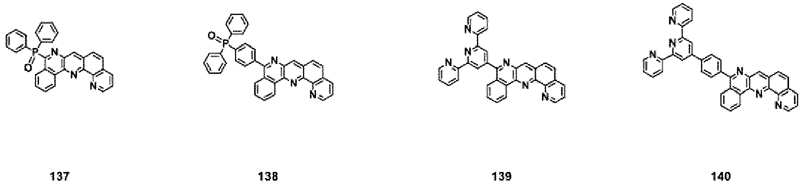

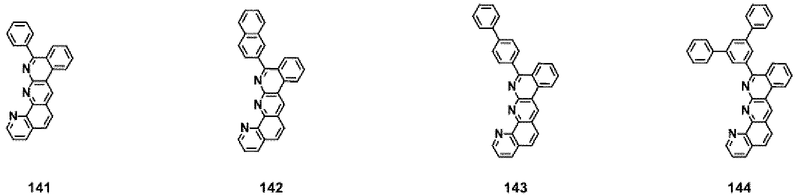

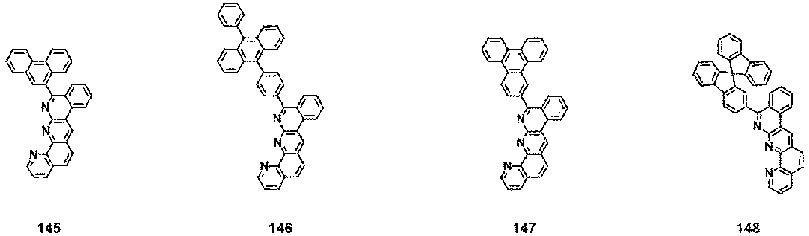

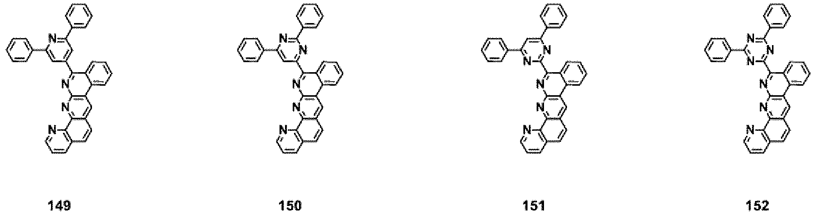

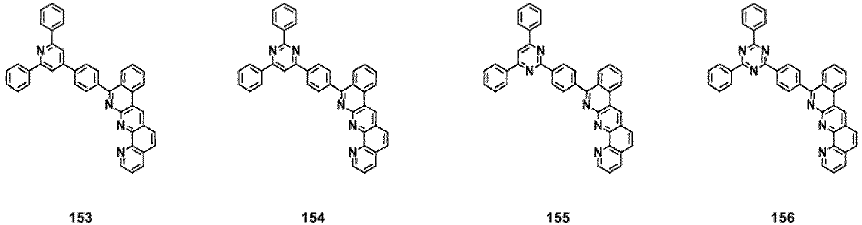

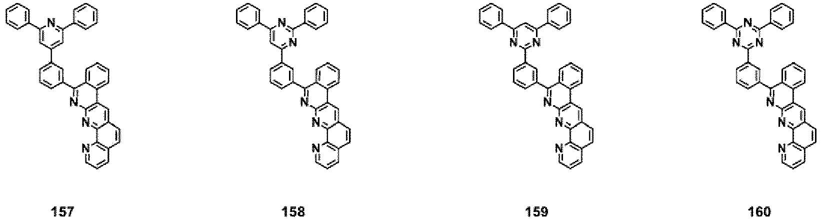

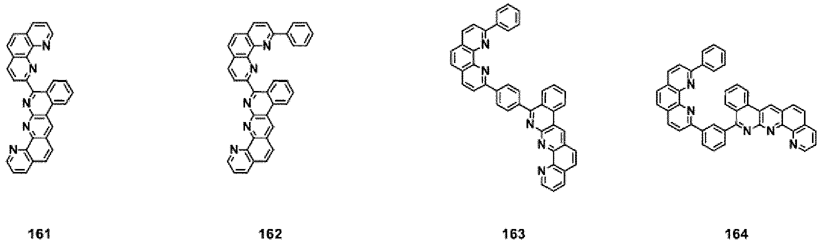

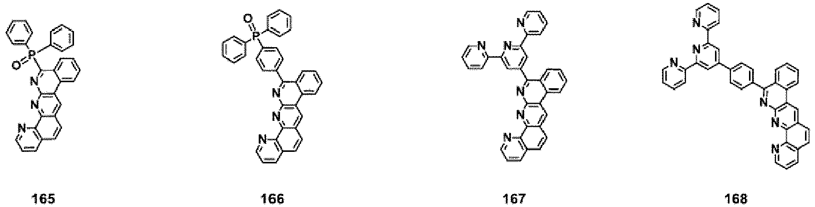

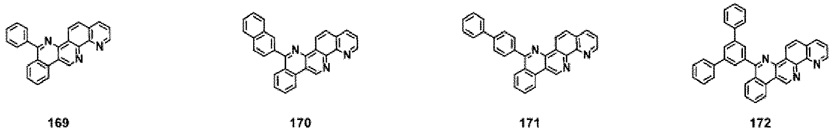

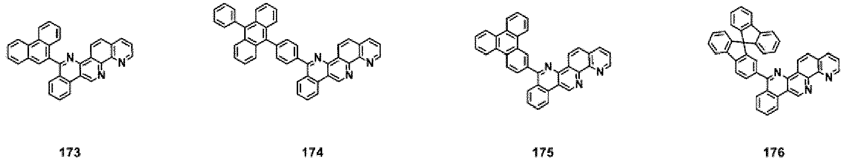

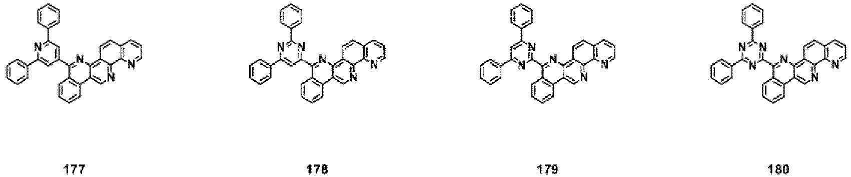

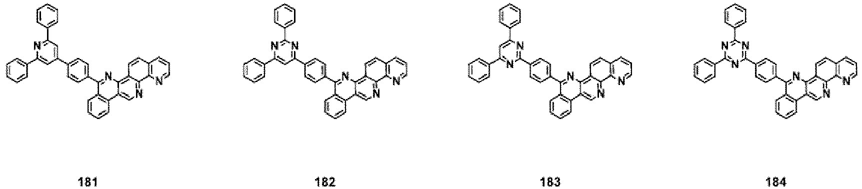

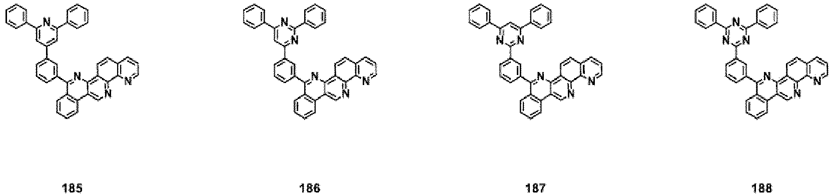

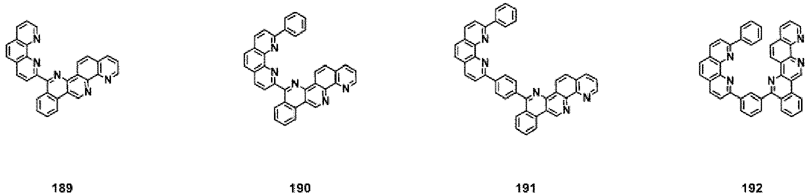

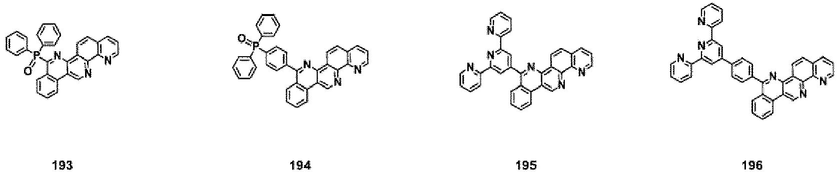

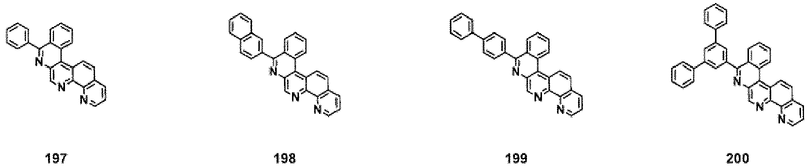

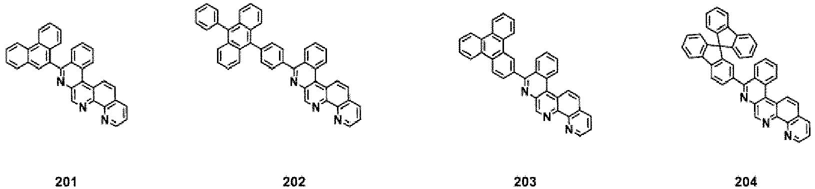

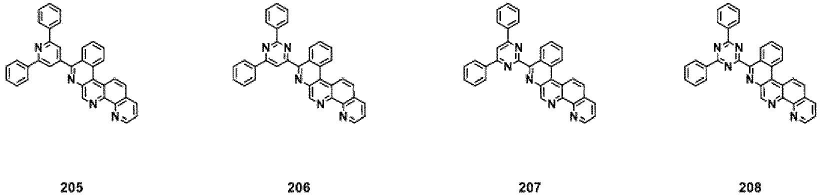

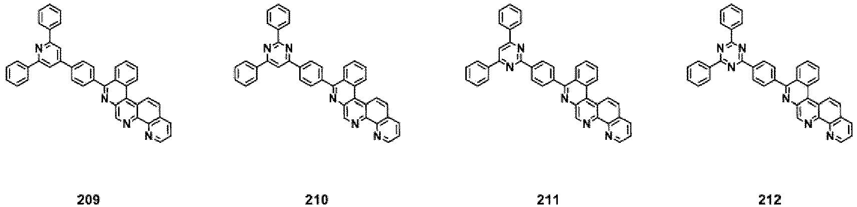

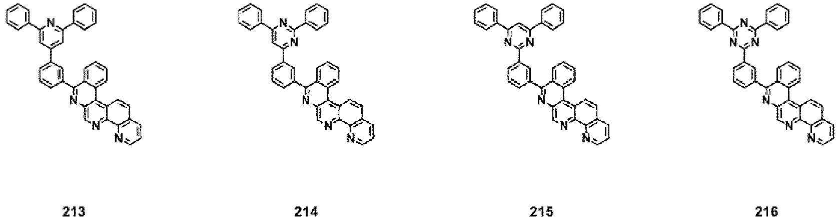

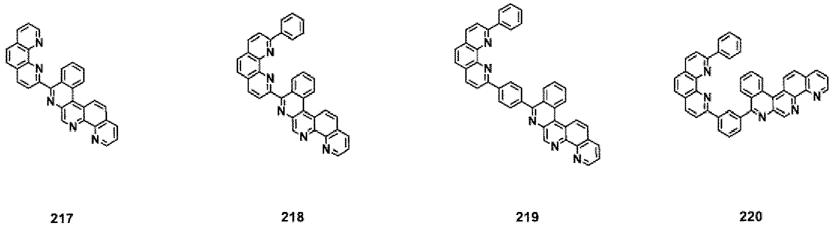

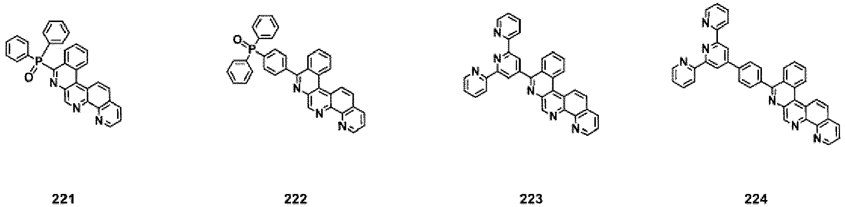

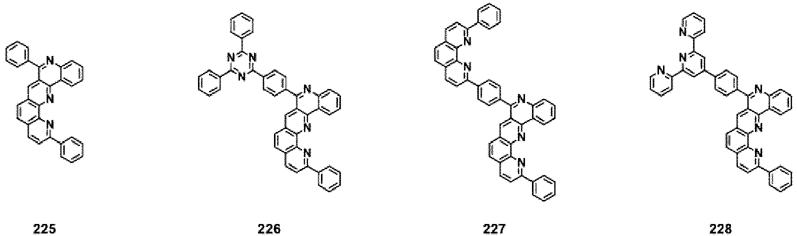

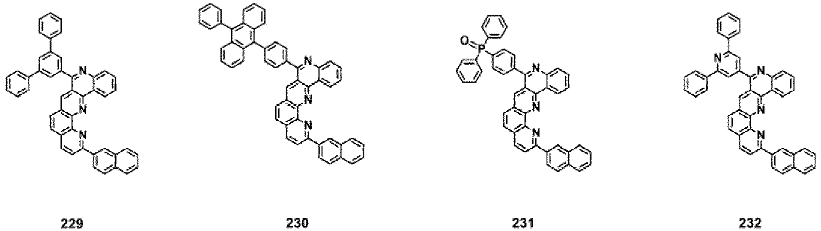

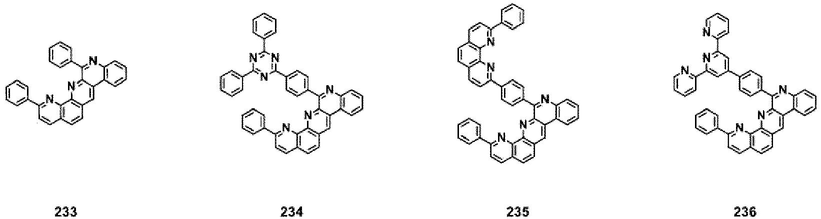

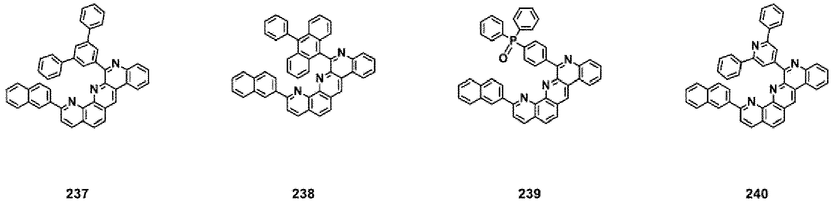

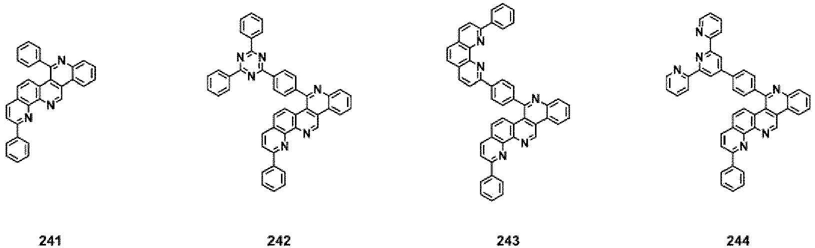

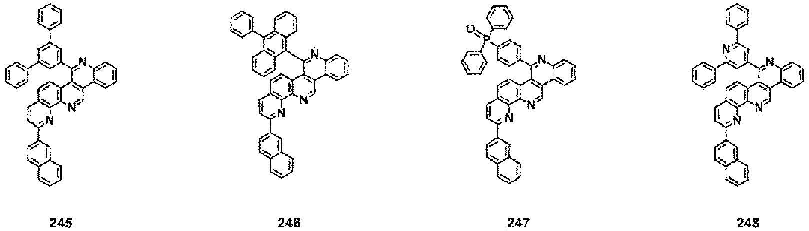

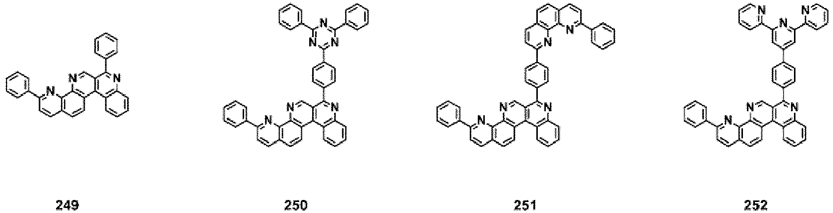

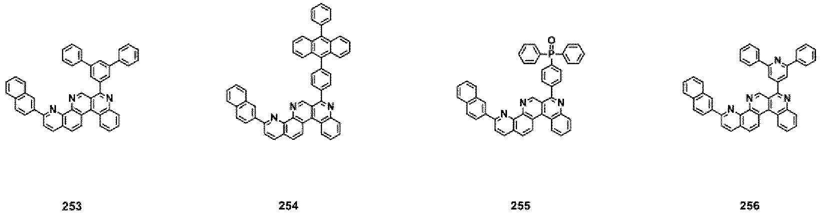

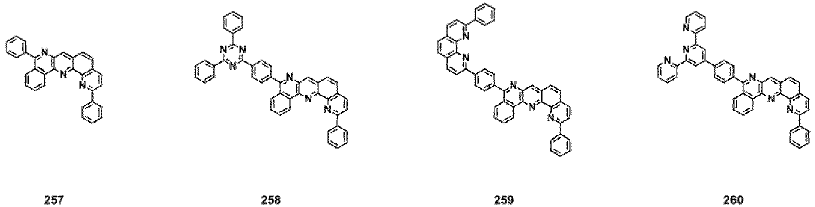

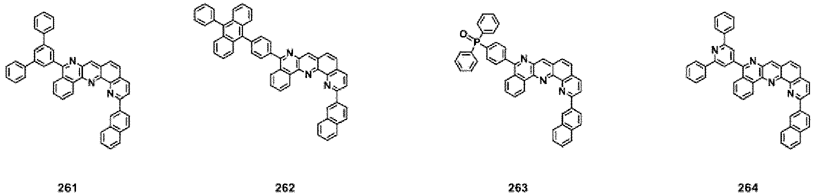

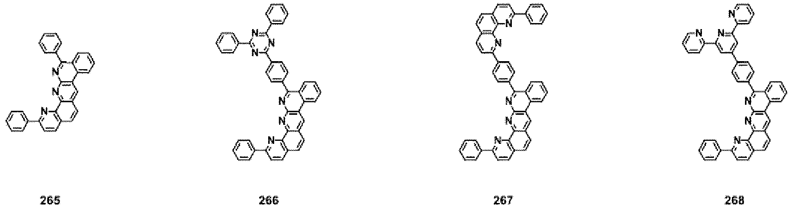

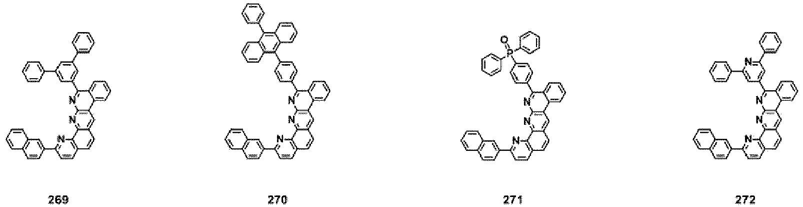

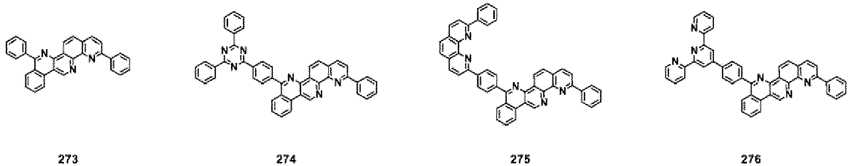

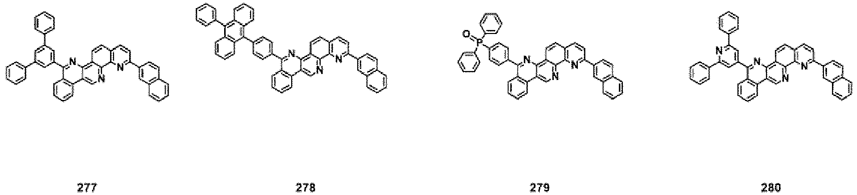

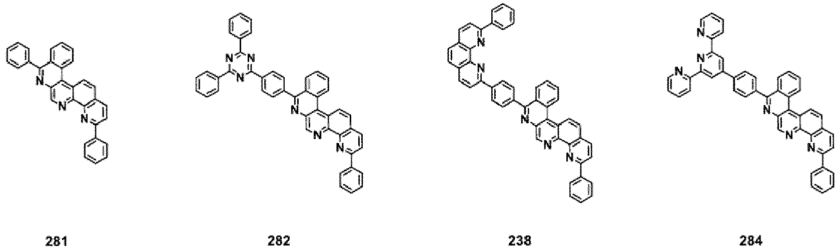

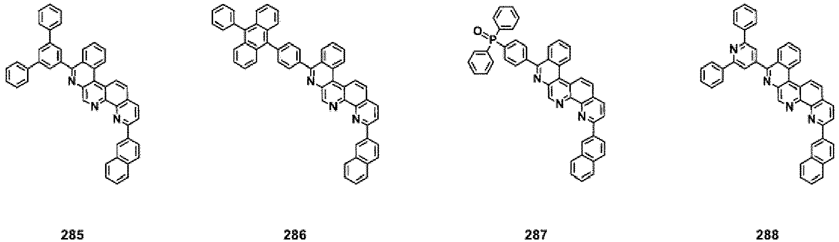

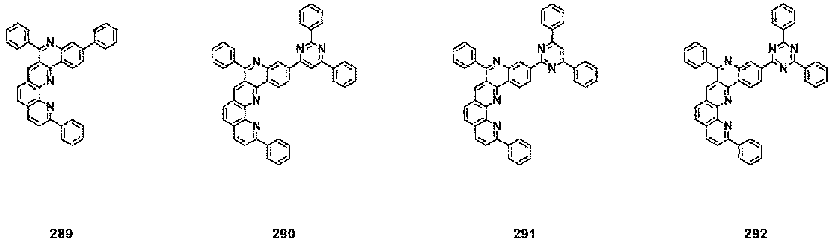

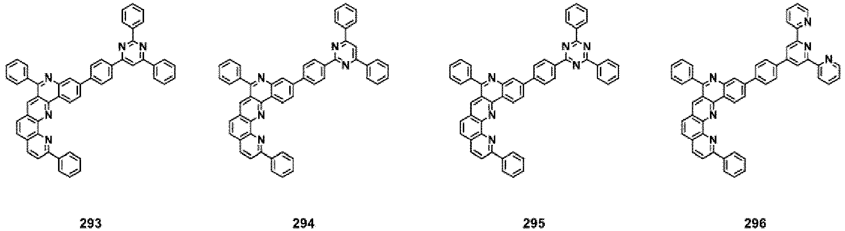

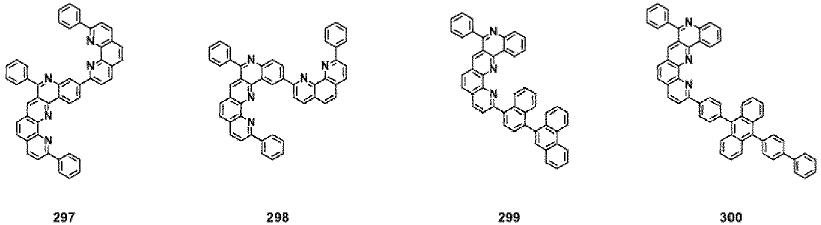

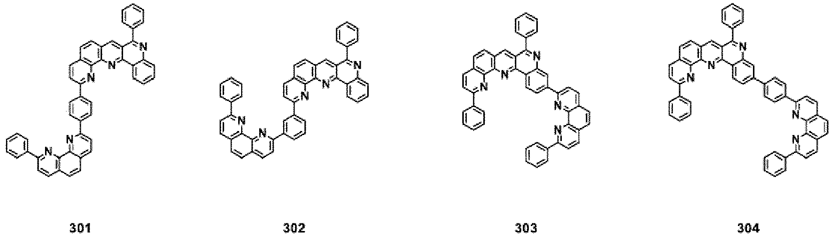

In addition, by introducing various substituents to the structure of Chemical Formula 1, compounds having unique properties of the introduced substituents may be synthesized. For example, by introducing substituents normally used as hole injection layer materials, hole transfer layer materials, light emitting layer materials, electron transfer layer materials and charge generation layer materials used for manufacturing an organic light emitting device to the core structure, materials satisfying conditions required for each organic material layer may be synthesized.

In addition, by introducing various substituents to the structure of Chemical Formula 1, the energy band gap may be finely controlled, and meanwhile, properties at interfaces between organic materials are enhanced, and material applications may become diverse.

One embodiment of the present specification provides an organic light emitting device including a first electrode; a second electrode; and one or more organic material layers provided between the first electrode and the second electrode, wherein one or more layers of the organic material layers include one or more types of the heterocyclic compound represented by Chemical Formula 1.

In one embodiment of the present specification, one or more layers of the organic material layers include one type of the heterocyclic compound represented by Chemical Formula 1.

In one embodiment of the present specification, the first electrode may be an anode, and the second electrode may be a cathode.

In another embodiment of the present specification, the first electrode may be a cathode, and the second electrode may be an anode.

In one embodiment of the present specification, the organic light emitting device may be a blue organic light emitting device, and the heterocyclic compound represented by Chemical Formula 1 may be used as a material of the blue organic light emitting device. For example, the heterocyclic compound represented by Chemical Formula 1 may be included in an electron transfer layer or a charge generation layer of the blue organic light emitting device.

In one embodiment of the present specification, the organic light emitting device may be a green organic light emitting device, and the heterocyclic compound represented by Chemical Formula 1 may be used as a material of the green organic light emitting device. For example, the heterocyclic compound represented by Chemical Formula 1 may be included in an electron transfer layer or a charge generation layer of the green organic light emitting device.

In one embodiment of the present specification, the organic light emitting device may be a red organic light emitting device, and the heterocyclic compound represented by Chemical Formula 1 may be used as a material of the red organic light emitting device. For example, the heterocyclic compound represented by Chemical Formula 1 may be included in an electron transfer layer or a charge generation layer of the red organic light emitting device.

The organic light emitting device of the present specification may be manufactured using common organic light emitting device manufacturing methods and materials except that one or more organic material layers are formed using the heterocyclic compound described above.

The compound may be formed into an organic material layer using a solution coating method as well as a vacuum deposition method when manufacturing the organic light emitting device. Herein, the solution coating method means spin coating, dip coating, inkjet printing, screen printing, a spray method, roll coating and the like, but is not limited thereto.

The organic material layer of the organic light emitting device of the present specification may be formed in a single layer structure, but may be formed in a multilayer structure in which two or more organic material layers are laminated. For example, the organic light emitting device of the present disclosure may have a structure including a hole injection layer, a hole transfer layer, a light emitting layer, an electron transfer layer, an electron injection layer and the like as the organic material layer. However, the structure of the organic light emitting device is not limited thereto, and may include a smaller number of organic material layers.

In the organic light emitting device of the present specification, the organic material layer includes an electron transfer layer, and the electron transfer layer may include the heterocyclic compound represented by Chemical Formula 1.

In the organic light emitting device of the present specification, the organic material layer includes a charge generation layer, and the charge generation layer may include the heterocyclic compound represented by Chemical Formula 1.

The organic light emitting device of the present disclosure may further include one, two or more layers selected from the group consisting of a light emitting layer, a hole injection layer, a hole transfer layer, an electron injection layer, an electron transfer layer, an electron blocking layer and a hole blocking layer.

FIG. 1 to FIG. 3 illustrate a lamination order of electrodes and organic material layers of the organic light emitting device according to one embodiment of the present specification. However, the scope of the present application is not limited to these diagrams, and structures of organic light emitting devices known in the art may also be used in the present application.

FIG. 1 illustrates an organic light emitting device in which an anode (200), an organic material layer (300) and a cathode (400) are consecutively laminated on a substrate (100). However, the structure is not limited to such a structure, and as illustrated in FIG. 2, an organic light emitting device in which a cathode, an organic material layer and an anode are consecutively laminated on a substrate may also be obtained.

FIG. 3 illustrates a case of the organic material layer being a multilayer. The organic light emitting device according to FIG. 3 includes a hole injection layer (301), a hole transfer layer (302), a light emitting layer (303), an electron transfer layer (304) and an electron injection layer (305). However, the scope of the present application is not limited to such a lamination structure, and as necessary, layers other than the light emitting layer may not be included, and other necessary functional layers may be further added.

The organic material layer including the heterocyclic compound represented by Chemical Formula 1 may further include other materials as necessary.

In addition, one embodiment of the present specification provides an organic light emitting device including a first electrode; a second electrode; and one or more organic material layers provided between the first electrode and the second electrode, wherein the organic material layer includes a first stack including a first light emitting layer, a charge generation layer provided on the first stack and a second stack provided on the charge generation layer and including a second light emitting layer, and the charge generation layer includes the heterocyclic compound represented by Chemical Formula 1.

In the organic light emitting device according to one embodiment of the present specification, the charge generation layer includes an N-type charge generation layer, and the N-type charge generation layer includes the heterocyclic compound represented by Chemical Formula 1.

In the organic light emitting device according to one embodiment of the present specification, the charge generation layer may further include a P-type charge generation layer.

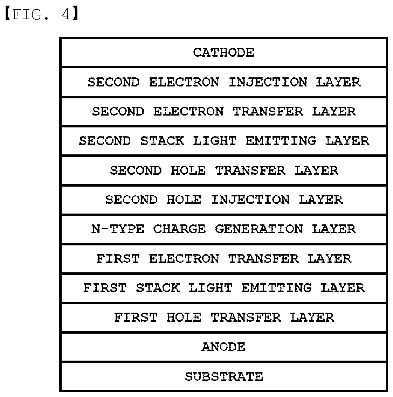

As the organic light emitting device according to one embodiment of the present specification, an organic light emitting device having a 2-stack tandem structure is illustrated in FIG. 4.

Herein, in the organic light emitting device having a 2-stack tandem structure of FIG. 4, a first hole injection layer, a first electron blocking layer, a first hole blocking layer, a second electron blocking layer, a second hole blocking layer, a P-type charge generation layer and the like may be added as necessary.

In the organic light emitting device according to one embodiment of the present specification, materials other than the heterocyclic compound represented by Chemical Formula 1 are illustrated below, however, these are for illustrative purposes only and not for limiting the scope of the present application, and these materials may be replaced by materials known in the art.

As the anode material, materials having relatively large work function may be used, and transparent conductive oxides, metals, conductive polymers or the like may be used. Specific examples of the anode material include metals such as vanadium, chromium, copper, zinc and gold, or alloys thereof; metal oxides such as zinc oxide, indium oxide, indium tin oxide (ITO) and indium zinc oxide (IZO); combinations of metals and oxides such as ZnO:Al or SnO₂:Sb; conductive polymers such as poly(3-methylthiophene), poly[3,4-(ethylene-1,2-dioxy)thiophene] (PEDOT), polypyrrole and polyaniline, and the like, but are not limited thereto.

As the cathode material, materials having relatively small work function may be used, and metals, metal oxides, conductive polymers or the like may be used. Specific examples of the cathode material include metals such as magnesium, calcium, sodium, potassium, titanium, indium, yttrium, lithium, gadolinium, aluminum, silver, tin and lead, or alloys thereof; multilayer structure materials such as LiF/Al or LiO₂/Al, and the like, but are not limited thereto.

As the hole injection material, known hole injection materials may be used, and for example, phthalocyanine compounds such as copper phthalocyanine disclosed in US Patent No. 4,356,429, or starburst-type amine derivatives such as tris(4-carbazoyl-9-ylphenyl)amine (TCTA), 4,4',4"-tri[phenyl(m-tolyl)amino]triphenylamine (m-MTDATA) or 1,3,5-tris[4-(3-methylphenylphenylamino)phenyl]benzene (m-MTDAPB) described in the literature [Advanced Material, 6, p.677 (1994)], polyaniline/dodecylbenzene sulfonic acid, poly(3,4-ethylenedioxythiophene)/poly(4-styrenesulfonate), polyaniline/camphor sulfonic acid or polyaniline/poly(4-styrenesulfonate) that are conductive polymers having solubility, and the like, may be used.

As the hole transfer material, pyrazoline derivatives, arylamine-based derivatives, stilbene derivatives, triphenyldiamine derivatives and the like may be used, and low molecular or high molecular materials may also be used.

As the electron transfer material, metal complexes of oxadiazole derivatives, anthraquinodimethane and derivatives thereof, benzoquinone and derivatives thereof, naphthoquinone and derivatives thereof, anthraquinone and derivatives thereof, tetracyanoanthraquinodimethane and derivatives thereof, fluorenone derivatives, diphenyldicyanoethylene and derivatives thereof, diphenoquinone derivatives, 8-hydroxyquinoline and derivatives thereof, and the like, may be used, and high molecular materials may also be used as well as low molecular materials.

As examples of the electron injection material, LiF is typically used in the art, however, the present application is not limited thereto.

As the light emitting material, red, green or blue light emitting materials may be used, and as necessary, two or more light emitting materials may be mixed and used. Herein, two or more light emitting materials may be used by being deposited as individual sources of supply or by being premixed and deposited as one source of supply. In addition, fluorescent materials may also be used as the light emitting material, however, phosphorescent materials may also be used. As the light emitting material, materials emitting light by bonding electrons and holes injected from an anode and a cathode, respectively, may be used alone, however, materials having a host material and a dopant material involving in light emission together may also be used.

When mixing light emitting material hosts, same series hosts may be mixed, or different series hosts may be mixed. For example, any two or more types of materials among n-type host materials or p-type host materials may be selected and used as a host material of a light emitting layer.

The organic light emitting device according to one embodiment of the present specification may be a top-emission type, a bottom-emission type or a dual-emission type depending on the materials used.

The compound according to one embodiment of the present specification may also be used in an organic electronic device including an organic solar cell, an organic photo conductor, an organic transistor and the like under a similar principle used in the organic light emitting device.

Hereinafter, the present specification will be described in more detail with reference to examples, however, these are for illustrative purposes only, and the scope of the present application is not limited thereto.

### [Preparation Example] Preparation of Compound 14

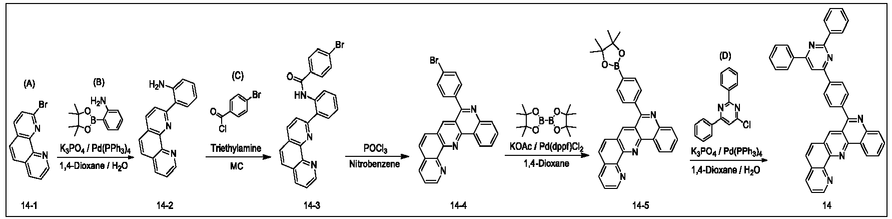

### 1) Preparation of Compound 14-2

1,4-Dioxane (400 ml) and H₂O (100 ml) were introduced to 2-bromo-1,10-phenanthroline (A) (20 g, 0.077 mol, 1 eq.), 2-(4,4,5,5-tetramethyl-1,3,2-dioxaborolan-2-yl)aniline (B) (20.3 g, 0.092 mol, 1.2 eq.), K₃PO₄ (49.1 g, 0.23 mol, 3 eq.) and Pd(PPh₃)₄ (tetrakis(triphenylphosphine)palladium(0)) (4.45 g, 0.0038 mol, 0.05 eq.), and stirred for 6 hours at 100°C. After terminating the reaction by introducing water thereto, the result was extracted using methylene chloride (MC) and water. After that, moisture was removed using MgSO₄, and the result was separated using a silica gel column to obtain Compound 14-2 (16 g) in a 76% yield.

### 2) Preparation of Compound 14-3

After dissolving Compound 14-2 (16 g, 0.058 mol, 1 eq.) and triethylamine (7.1 g, 0.70 mol, 1.2 eq.) in MC (160 ml), 4-bromobenzoyl chloride **(C)** (15.5 g, 0.092 mol, 1.2 eq.) was slowly introduced thereto at 0°C, and the result was stirred for 4 hours at room temperature (RT). After terminating the reaction by introducing water thereto, the result was extracted using MC and water. After that, moisture was removed using MgSO₄, and the result was separated using a silica gel column to obtain Compound 14-3 (18 g) in a 67% yield.

### 3) Preparation of Compound 14-4

Nitrobenzene (180 ml) was introduced to Compound 14-3 (18 g, 0.039 mol, 1 eq.) and POCl₃ (18.2 g, 0.118 mol, 3 eq.), and stirred for 12 hours at 160°C. After terminating the reaction by introducing water thereto, the result was extracted using MC and water. After that, moisture was removed using MgSO₄, and the result was separated using a silica gel column to obtain Compound 14-4 (11 g) in a 64% yield.

### 4) Preparation of Compound 14-5

1,4-Dioxane (110 ml) was introduced to Compound 14-4 (11 g, 0.025 mol, 1 eq.), 4,4,4',4',5,5,5',5'-octamethyl-2,2'-bi(1,3,2-dioxaborolane) (9.6 g, 0.038 mol, 1.5 eq.), KOAc (potassium acetate) (7.4 g, 0.075 mol, 3 eq.) and Pd(dppf)Cl₂ ([1,1'-bis(diphenylphosphino)ferrocene]dichloropalladium (II)) (1.8 g, 0.0025 mol, 0.1 eq.), and stirred for 8 hours at 100°C. After terminating the reaction by introducing water thereto, the result was extracted using MC and water. After that, moisture was removed using MgSO₄, and the result was separated using a silica gel column to obtain Compound 14-5 (10 g) in a 82% yield.

### 5) Preparation of Compound 14

1,4-Dioxane (200 ml) and H₂O (50 ml) were introduced to Compound 14-5 (10 g, 0.02 mol, 1 eq.), 4-chloro-2,6-diphenylpyrimidine **(D)** (5.8 g, 0.021 mol, 1.05 eq.), K₃PO₄ (13.1 g, 0.062 mol, 3 eq.) and Pd(PPh₃)₄ (1.19 g, 0.001 mol, 0.05 eq.), and stirred for 6 hours at 100°C. After terminating the reaction by introducing water thereto, the result was extracted using MC and water. After that, moisture was removed using MgSO₄, and the result was separated using a silica gel column to obtain Compound 14 (9 g) in a 74% yield.

Compounds were synthesized in the same manner as in the preparation example except that Intermediates A, B, C and D of the following Table 1 were used instead of 2-bromo-1,10-phenanthroline (A), 2-(4,4,5,5-tetramethyl-1,3,2-dioxaborolan-2-yl)aniline (B), 4-bromobenzoyl chloride (C) and 4-chloro-2,6-diphenylpyrimidine (D).

**[Table 1]**

| Compou nd No. | Intermediat e A | Intermediat e B | Intermediat e C | Intermediat e D | Yiel d |
|---|---|---|---|---|---|
| 1 | 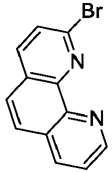 | 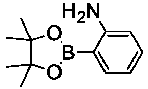 | 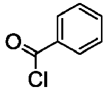 | - | 43% |
| 3 | 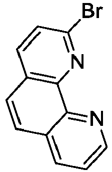 | 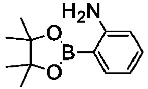 | 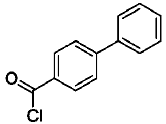 | - | 40% |
| 15 | 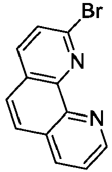 | 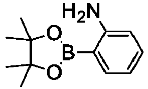 | 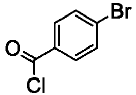 | 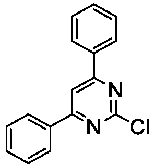 | 49% |
| 24 | 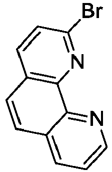 | 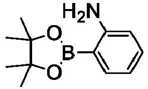 | 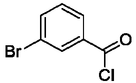 | 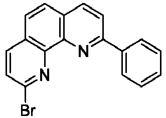 | 50% |
| 28 | 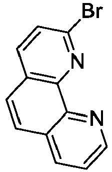 | 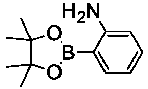 | 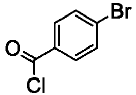 | 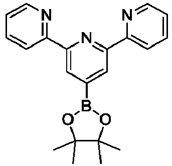 | 48% |
| 58 | 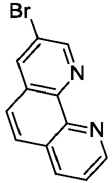 | 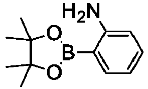 | 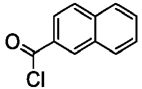 | - | 38% |
| 63 | 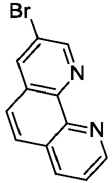 | 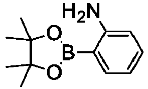 | 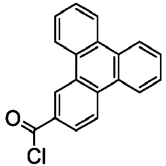 | - | 41% |
| 72 | 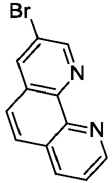 | 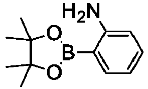 | 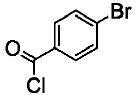 | 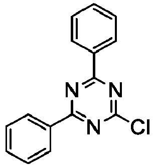 | 50% |
| 88 | 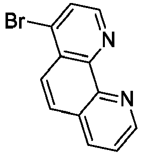 | 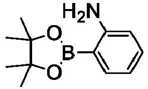 | 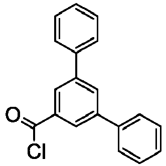 | - | 37% |
| 98 | 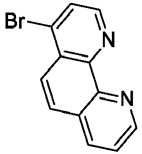 | 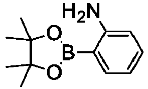 | 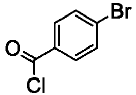 | 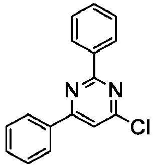 | 44% |
| 113 | 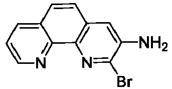 | 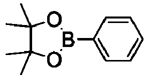 | 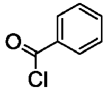 | - | 54% |
| 146 | 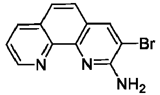 | 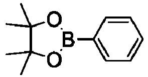 | 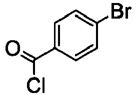 | 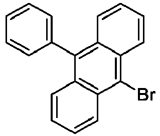 | 50% |
| 186 | 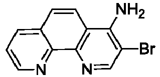 | 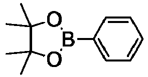 | 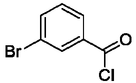 | 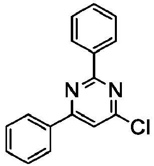 | 48% |
| 199 | 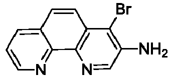 | 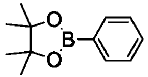 | 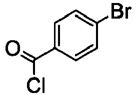 | 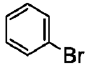 | 49% |
| 225 | 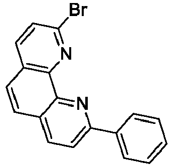 | 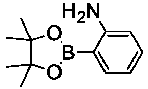 | 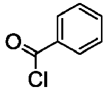 | - | 57% |
| 226 | 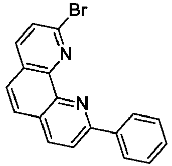 | 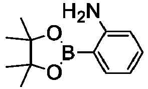 | 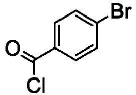 | 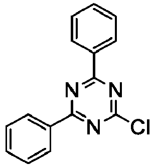 | 53% |
| 289 | 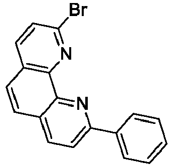 | 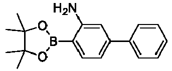 | 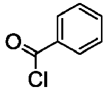 | - | 60% |
| 297 | 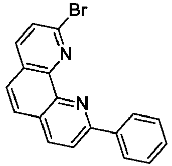 | 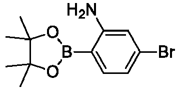 | 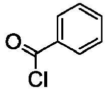 | 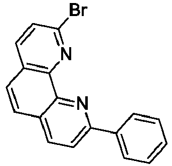 | 50 |

Compounds were prepared in the same manner as in the preparation example, and the synthesis identification results are shown in Table 2 and Table 3. Table 2 shows measurement values of ¹H NMR (CDCl₃, 200 Mz), and Table 3 shows measurement values of FD-mass spectrometry (FD-MS: field desorption mass spectrometry).

**[Table 2]**

| Compou nd | ¹H NMR (CDCl₃, 200 Mz) |
|---|---|
| 1 | δ=8.83 (1H, d), 8.30(5H, m), 8.06(2H, dd), 7.78(2H, m), 7.54(5H, m) |
| 3 | δ=8.81(3H, m), 8.38(1H, d), 8.23(2H, m), 8.06(1H, d), 7.98(1H, d) 7.78(4H, m), 7.51(7H, m) |
| 14 | δ=8.81(3H, m), 8.23(8H, m), 8.06(1H, d), 7.98(1H, d) 7.78(4H, m), 7.51(8H, m) |
| 15 | δ=8.81(3H, m), 8.38(1H, d), 8.23(3H, m), 8.06(1H, d), 7.98(1H, d), 7.78(8H, m), 7.51(8H, m) |
| 24 | δ=8.83(1H, d), 8.72(1H, s), 8.30(7H, m), 8.06(5H, m), 7.81(3H, m), 7.54(6H, m), 7.35(2H, m) |
| 28 | δ=9.30(2H, d), 9.15(2H, s), 8.81(3H, m), 8.53(2H, m), 8.38(1H, d), 8.22(2H, m), 8.06(1H, d), 7.98(1H, d), 7.60(8H, m), 7.14(2H, t) |
| 58 | δ=9.39 (1H, s), 8.85(2H, m), 8.38(2H, m), 8.00(6H, m), 7.78(2H, m), 7,60(4H, m) |
| 63 | δ=9.66(1H, s), 9.39(1H, s), 8.93(2H, d), 8.83(1H, d), 8.55(1H, d), 8.38(1H, d), 8.21(1H, d), 7.88(11H, m) 7.60(2H, m) |
| 72 | δ=9.39(1H, s), 8.81(3H, m), 8.38(1H, d), 8.28(4H, m), 7.98(3H, m), 7.88(4H, m), 7.41(8H, m) |
| 88 | δ=8.83(1H, d), 8.38(2H, m), 8.17(2H, s), 8.06(2H, m), 7.98(1H, d), 7.72(3H, m), 7.51(12H, m) |
| 98 | δ=8.83(3H, m), 8.23(7H, m), 8.06(2H, m), 7.98(1H, d), 7.79(4H, m), 7.51(8H, m) |
| 113 | δ=8.83(1H, d), 8.30(4H, m), 8.06(1H, d), 7.92(1H, d), 7.76(2H, m), 7.54(6H, m) |
| 146 | δ=8.81(3H, m), 8.38(1H, d), 8.17(1H, s), 8.06(1H, d), 7.91(5H, m), 7.76(2H, m), 7.51(12H, m), 7.28(2H, m) |
| 186 | δ=9.45(1H, s), 8.83(1H, d), 8.38(1H, d), 8.28(5H, m), 8.06(1H, d), 7.92(1H, d), 7.81(5H, m), 7.51(10H, m) |
| 199 | δ=9.15(1H, s), 8.83(3H, m), 8.38(1H, d), 8.06(1H, d), 7.88(5H, m), 7.51(8H, m) |
| 225 | δ=8.30(6H, m), 8.10 (2H, m), 7.98(1H, d), 7.78(2H, m), 7.54(7H, m) 7.35(1H, d) |
| 226 | δ=8.81(2H, d), 8.28 (8H, m), 8.10(2H, m), 7.98(1H, d), 7.81(4H, m), 7.51(11H, m) |
| 289 | δ=8.30(6H, m), 8.03(4H, m), 7.81(1H, d), 7.41(12H, m) |
| 297 | δ=8.78(1H, s), 8.54(1H, d), 8.30(6H, m), 8.23(1H, s), 8.06 (6H, m), 7.81(2H, m), 7.54(9H, m), 7.35(3H, m) |

**[Table 3]**

| Comp ound | FD-MS | Compo und | FD-MS |
|---|---|---|---|
| 1 | m/z=357.41 (C25H15N3=357.13) | 3 | m/z=433.50 (C31H19N3=433.16) |
| 14 | m/z=587.67 (C41H25N5=587.21) | 15 | m/z=587.67 (C41H25N5=587.21) |
| 24 | m/z=611.69 (C43H25N5=611.21) | 28 | m/z=588.66 (C40H24N6=588.21) |
| 58 | m/z=407.47 (C29H17N3=407.14) | 63 | m/z=507.58 (C37H21N3=507.17) |
| 72 | m/z=588.66 (C40H24N6=588.21) | 88 | m/z=509.19 (C37H23N3=509.60) |
| 98 | m/z=587.67 (C41H25N5=587.21) | 113 | m/z=357.41 (C25H15N3=357.13) |
| 146 | m/z=609.72 (C45H27N3=609.22) | 186 | m/z=587.67 (C41H25N5=587.21) |
| 199 | m/z=433.50 (C31H19N3=533.16) | 225 | m/z=433.50 (C31H19N3=433.16) |
| 226 | m/z=664.75 (C46H28N6=664.24) | 289 | m/z=509.60 (C37H23N3=509.19) |
| 297 | m/z=687.79 (C49H29N5=687.24) | | |

### [Experimental Example]

### <Experimental Example 1>

### 1) Manufacture of Organic Light Emitting Device

### Comparative Example 1-1

A transparent indium tin oxide (ITO) electrode thin film obtained from glass for an OLED (manufactured by Samsung-Corning Co., Ltd.) was ultrasonic cleaned using trichloroethylene, acetone, ethanol and distilled water consecutively for 5 minutes each, stored in isopropanol, and used. Next, the ITO substrate was installed in a substrate folder of a vacuum deposition apparatus, and the following 4,4',4"-tris(N,N-(2-naphthyl)-phenylamino)triphenylamine (2-TNATA) was introduced to a cell in the vacuum deposition apparatus.

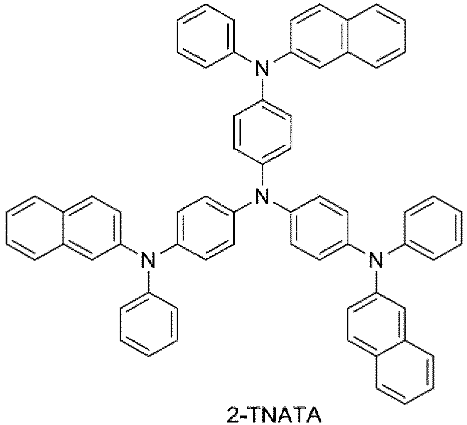

Subsequently, the chamber was evacuated until the degree of vacuum therein reached 10⁻⁶ torr, and then 2-TNATA was evaporated by applying a current to the cell to deposit a hole injection layer having a thickness of 600 Å on the ITO substrate. To another cell in the vacuum deposition apparatus, the following N,N'-bis(α-naphthyl)-N,N'-diphenyl-4,4'-diamine (NPB) was introduced, and evaporated by applying a current to the cell to deposit a hole transfer layer having a thickness of 300 Å on the hole injection layer.

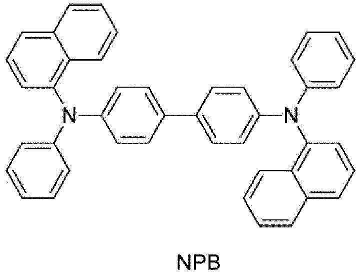

After forming the hole injection layer and the hole transfer layer as above, a blue light emitting material having a structure as below was deposited thereon as a light emitting layer. Specifically, in one side cell in the vacuum deposition apparatus, H1, a blue light emitting host material, was vacuum deposited to a thickness of 200 Å, and D1, a blue light emitting dopant material, was vacuum deposited thereon by 5% with respect to the host material.

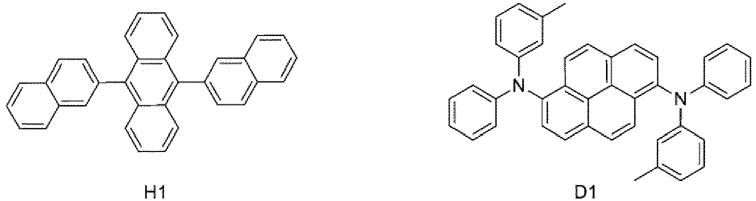

Subsequently, a compound of the following Structural Formula E1 was deposited to a thickness of 300 Å as an electron transfer layer.

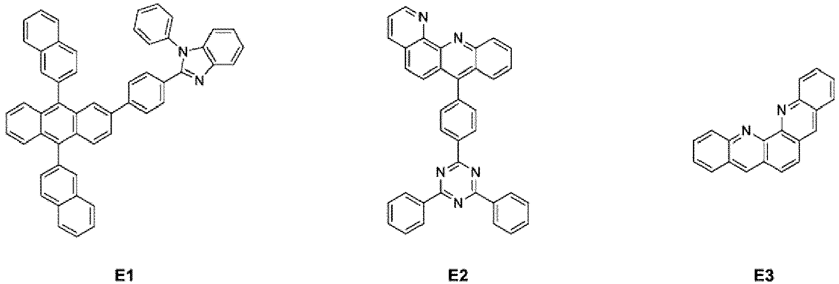

As an electron injection layer, lithium fluoride (LiF) was deposited to a thickness of 10 Å, and an Al cathode was employed to a thickness of 1,000 Å, and as a result, an OLED was manufactured. Meanwhile, all the organic compounds required to manufacture the OLED were vacuum sublimation purified under 10⁻⁸ torr to 10⁻⁶ torr by each material to be used in the OLED manufacture.

### Comparative Examples 1-2 and 1-3 and Examples 1-1 to 1-19

Organic electroluminescent devices were manufactured in the same manner as in Comparative Example 1-1 except that compounds shown in the following Table 4 were each used instead of E1 used when forming the electron transfer layer.

### 2) Measurement of Driving Voltage and Light Emission Efficiency of Organic Light Emitting Device

For each of the organic electroluminescent devices manufactured as above, electroluminescent (EL) properties were measured using M7000 manufactured by McScience Inc., and with the measurement results, T₉₅ was measured when standard luminance was 3,500 cd/m² through a lifetime measurement system (M6000) manufactured by McScience Inc.. Results of measuring driving voltage, light emission efficiency, color coordinate (CIE) and lifetime of the blue organic light emitting devices manufactured according to the present disclosure are as shown in Table 4.

**[Table 4]**

| | Compou nd | Driving Voltage (V) | Light Emission Efficienc y (cd/A) | CIE (x, y) | Lifetim e (T₉₅) |
|---|---|---|---|---|---|
| Example 1-1 | 1 | 4.90 | 6.69 | (0.134, 0.100) | 49 |
| Example 1-2 | 3 | 4.86 | 6.80 | (0.134, 0.101) | 47 |
| Example 1-3 | 14 | 4.79 | 6.77 | (0.134, 0.101) | 46 |
| Example 1-4 | 15 | 4.85 | 6.73 | (0.134, 0.100) | 46 |
| Example 1-5 | 24 | 4.87 | 6.83 | (0.134, 0.101) | 51 |
| Example 1-6 | 28 | 4.80 | 6.85 | (0.134, 0.100) | 42 |
| Example 1-7 | 58 | 4.93 | 6.81 | (0.134, 0.100) | 45 |
| Example 1-8 | 63 | 4.90 | 6.76 | (0.134, 0.101) | 49 |
| Example 1-9 | 72 | 4.94 | 6.70 | (0.134, 0.100) | 41 |
| Example 1-10 | 88 | 4.78 | 6.68 | (0.134, 0.100) | 41 |
| Example 1-11 | 98 | 4.89 | 6.80 | (0.134, 0.100) | 46 |
| Example 1-12 | 113 | 4.93 | 6.89 | (0.134, 0.101) | 50 |
| Example 1-13 | 146 | 4.83 | 6.79 | (0.134, 0.100) | 51 |
| Example 1-14 | 186 | 4.91 | 6.63 | (0.134, 0.101) | 47 |
| Example 1-15 | 199 | 4.82 | 6.82 | (0.134, 0.100) | 46 |
| Example 1-16 | 225 | 4.97 | 6.70 | (0.134, 0.101) | 44 |
| Example 1-17 | 226 | 4.88 | 6.88 | (0.134, 0.100) | 47 |
| Example 1-18 | 289 | 4.90 | 6.77 | (0.134, 0.100) | 48 |
| Example 1-19 | 297 | 4.81 | 6.66 | (0.134, 0.101) | 50 |
| Comparative Example 1-1 | E1 | 5.57 | 6.13 | (0.134, 0.100) | 34 |
| Comparative Example 1-2 | E2 | 5.50 | 6.20 | (0.134, 0.101) | 35 |
| Comparative Example 1-3 | E3 | 5.84 | 5.99 | (0.134, 0.100) | 33 |

As seen from the results of Table 4, the organic light emitting device using the electron transfer layer material of the blue organic light emitting device of the present disclosure had lower driving voltage and significantly improved light emission efficiency and lifetime compared to Comparative Examples 1-1 to 1-3.

Such a result is considered to be due to the fact that, when using the compound having proper length and strength, and flatness as an electron transfer layer, a compound in an excited state is made by receiving electrons under a specific condition, and particularly when an excited state is formed in the hetero-skeleton site of the compound, excited energy will move to a stable state before the excited hetero-skeleton site goes through other reactions, and as a result, the relatively stabilized compound is capable of efficiently transferring electrons without the compound being decomposed or destroyed. For reference, those that are stable when excited are considered to be aryl or acene-based compounds or polycyclic hetero-compounds.

Accordingly, it is considered that excellent results were obtained in all aspects of driving voltage, light emission efficiency and lifetime by the compound of the present disclosure enhancing enhanced electron-transfer properties or improved stability.

### <Experimental Example 2>

### 1) Manufacture of Organic Light Emitting Device

### Examples 2-1 to 2-19 and Comparative Examples 2-1 to 2-3

A glass substrate on which ITO was coated as a thin film to a thickness of 1,500 Å was cleaned with distilled water ultrasonic waves. After the cleaning with distilled water was finished, the substrate was ultrasonic cleaned with solvents such as acetone, methanol and isopropyl alcohol, then dried, and UVO treatment was conducted for 5 minutes using UV in a UV cleaner. After that, the substrate was transferred to a plasma cleaner (PT), and after conducting plasma treatment under vacuum for ITO work function and residual film removal, the substrate was transferred to a thermal deposition apparatus for organic deposition.

On the transparent ITO electrode (anode), organic materials were formed in a 2-stack white organic light emitting device (WOLED) structure. As for the first stack, TAPC was thermal vacuum deposited first to a thickness of 300 Å to form a hole transfer layer. After forming the hole transfer layer, a light emitting layer was thermal vacuum deposited thereon as follows. The light emitting layer was deposited to 300 Å by doping TCz1, a host, with FIrpic, a blue phosphorescent dopant, by 8%. After forming an electron transfer layer to 400 Å using TmPyPB, a compound described in the following Table 5 was doped with Cs₂CO₃ by 20% to form a charge generation layer to 100 Å.

As for the second stack, MoO₃ was thermal vacuum deposited first to a thickness of 50 Å to form a hole injection layer. A hole transfer layer that is a common layer was formed to 100 Å by doping MoO₃ to TAPC by 20%, and then depositing TAPC to 300 Å. A light emitting layer was deposited to 300 Å thereon by doping TCz1, a host, with Ir(ppy)₃, a green phosphorescent dopant, by 8%, and an electron transfer layer was formed to 600 Å using TmPyPB. Lastly, an electron injection layer was formed on the electron transfer layer by depositing lithium fluoride (LiF) to a thickness of 10 Å, and then a cathode was formed on the electron injection layer by depositing an aluminum (Al) cathode to a thickness of 1,200 Å, and as a result, an organic electroluminescent device was manufactured.

Meanwhile, all the organic compounds required to manufacture the OLED were vacuum sublimation purified under 10⁻⁸ torr to 10⁻⁶ torr for each material to be used in the OLED manufacture.

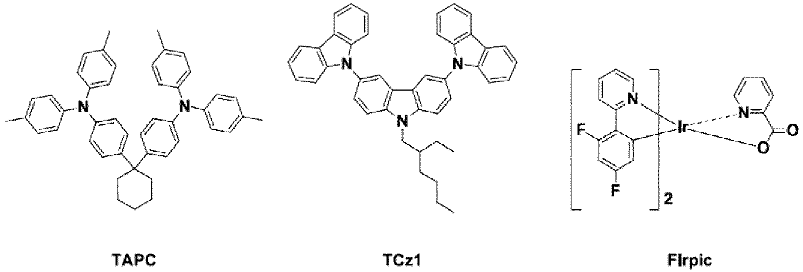

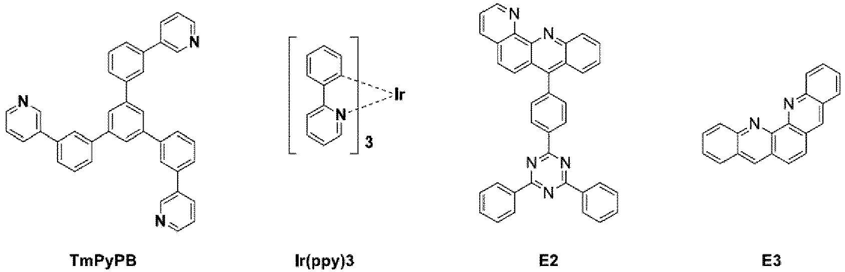

### 2) Measurement of Driving Voltage and Light Emission Efficiency of Organic Light Emitting Device

For each of the organic electroluminescent devices manufactured as above, electroluminescent (EL) properties were measured using M7000 manufactured by McScience Inc., and with the measurement results, T₉₅ was measured when standard luminance was 3,500 cd/m² through a lifetime measurement system (M6000) manufactured by McScience Inc.. Results of measuring driving voltage, light emission efficiency, external quantum efficiency and color coordinate (CIE) of the white organic electroluminescent devices manufactured according to the present disclosure are as shown in Table 5.

**[Table 5]**

| | Compoun d | Driving Voltage (V) | Light Emission Efficienc y (cd/A) | CIE (x, y) | Lifeti me (T₉₅) |
|---|---|---|---|---|---|
| Example 2-1 | 1 | 7.32 | 62.78 | (0.210, 0.414) | 83 |
| Example 2-2 | 3 | 7.66 | 63.66 | (0.218, 0.420) | 79 |
| Example 2-3 | 14 | 7.39 | 62.72 | (0.217, 0.425) | 70 |
| Example 2-4 | 15 | 7.11 | 66.29 | (0.210, 0.427) | 88 |
| Example 2-5 | 24 | 7.54 | 63.11 | (0.213, 0.421) | 91 |
| Example 2-6 | 28 | 7.07 | 68.23 | (0.206, 0.428) | 77 |
| Example 2-7 | 58 | 7.25 | 63.88 | (0.205, 0.433) | 83 |
| Example 2-8 | 63 | 7.45 | 60.58 | (0.219, 0.416) | 72 |
| Example 2-9 | 72 | 7.56 | 69.01 | (0.209, 0.425) | 79 |
| Example 2-10 | 88 | 7.53 | 66.74 | (0.205, 0.427) | 70 |
| Example 2-11 | 98 | 7.40 | 61.82 | (0.207, 0.411) | 88 |
| Example 2-12 | 113 | 7.24 | 62.75 | (0.221, 0.435) | 93 |
| Example 2-13 | 146 | 7.44 | 63.45 | (0.218, 0.429) | 95 |
| Example 2-14 | 186 | 7.39 | 64,51 | (0.211, 0,426) | 92 |
| Example 2-15 | 199 | 7.37 | 66.02 | (0.216, 0.420) | 87 |
| Example 2-16 | 225 | 7.40 | 63.52 | (0.209, 0.425) | 89 |
| Example 2-17 | 226 | 7.33 | 66.18 | (0.208, 0.433) | 80 |
| Example 2-18 | 289 | 7.17 | 65.90 | (0.211, 0.428) | 76 |
| Example 2-19 | 297 | 7.22 | 67.63 | (0.217, 0.430) | 84 |
| Comparative Example 2-1 | TmPyPB | 8.24 | 55.65 | (0.210, 0.432) | 57 |
| Comparative Example 2-2 | E2 | 8.02 | 56.60 | (0.212, 0,434) | 60 |
| Comparative Example 2-3 | E3 | 8.22 | 55.72 | (0.211, 0.427) | 58 |

As seen from the results of Table 5, the organic electroluminescent device using the charge generation layer material of the 2-stack white organic electroluminescent device of the present disclosure had lower driving voltage, and improved lifetime and light emission efficiency compared to Comparative Examples 2-1 to 2-3.

Such a result is considered to be due to the fact that the compound of the present disclosure used as an N-type charge generation layer formed with the disclosed skeleton having proper length and strength, and flatness and a proper hetero-compound capable of binding to metals forms a gap state in the N-type charge generation layer by doping an alkali metal or an alkaline earth metal thereto, and electrons produced from a P-type charge generation layer are readily injected into an electron transfer layer through the gap state produced in the N-type charge generation layer. Accordingly, the P-type charge generation layer may favorably inject and transfer electrons to the N-type charge generation layer, and as a result, driving voltage was lowered, and efficiency and lifetime were improved in the organic light emitting device.

## Claims

1. A heterocyclic compound of the following Chemical Formula 1: 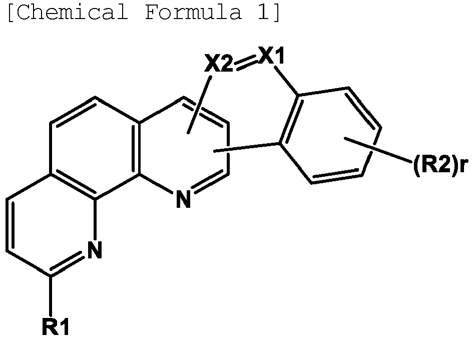
wherein, in Chemical Formula 1,
X1 and X2 are each independently N; or CR, and at least one of X1 and X2 is N,
R, R1 and R2 are each independently hydrogen; deuterium; a halogen group; a cyano group; a substituted or unsubstituted silyl group; a substituted or unsubstituted phosphine oxide group; a substituted or unsubstituted C1 to C60 alkyl group; a substituted or unsubstituted C3 to C60 cycloalkyl group; a substituted or unsubstituted C6 to C60 aryl group; or a substituted or unsubstituted C2 to C60 heteroaryl group, and
r is an integer of 1 to 4, and when 2 or greater, R2s are the same as or different from each other.

2. The heterocyclic compound of Claim 1, wherein Chemical Formula 1 is represented by the following Chemical Formula 1-1 or Chemical Formula 1-2: 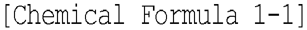 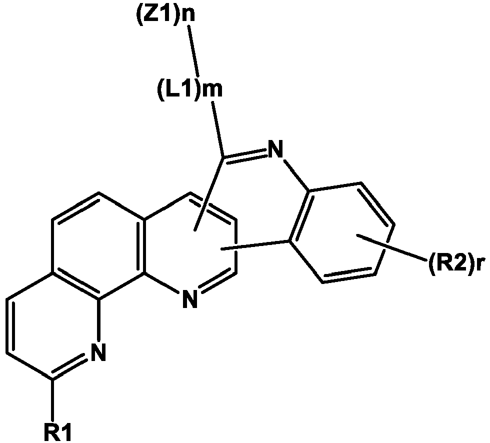 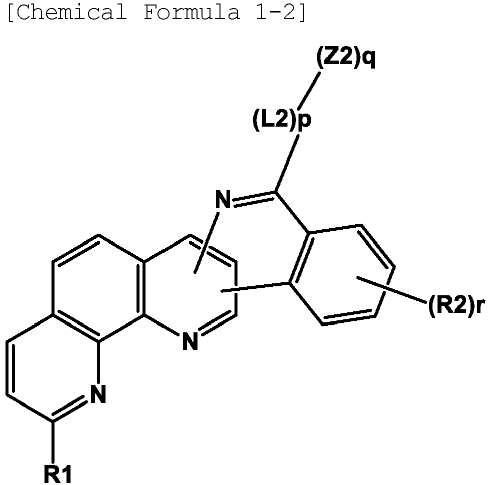
in Chemical Formulae 1-1 and 1-2,
R1, R2 and r have the same definitions as in Chemical Formula 1,
L1 and L2 are each independently a direct bond; a substituted or unsubstituted C6 to C60 arylene group; or a substituted or unsubstituted C2 to C60 heteroarylene group,
Z1 and Z2 are each independently a halogen group; a cyano group; a substituted or unsubstituted silyl group; a substituted or unsubstituted phosphine oxide group; a substituted or unsubstituted C1 to C60 alkyl group; a substituted or unsubstituted C3 to C60 cycloalkyl group; a substituted or unsubstituted C6 to C60 aryl group; or a substituted or unsubstituted C2 to C60 heteroaryl group,
m is an integer of 1 to 5, and when 2 or greater, L1s are the same as or different from each other,
n is an integer of 1 to 5, and when 2 or greater, Z1s are the same as or different from each other,
p is an integer of 1 to 5, and when 2 or greater, L2s are the same as or different from each other, and
q is an integer of 1 to 5, and when 2 or greater, Z2s are the same as or different from each other.

3. The heterocyclic compound of Claim 2, wherein Z1 and Z2 are each independently a substituted or unsubstituted phosphine oxide group; a substituted or unsubstituted C6 to C60 aryl group; or a substituted or unsubstituted C2 to C60 heteroaryl group.

4. The heterocyclic compound of Claim 1, wherein Chemical Formula 1 is represented by any one of the following Chemical Formula 4-1 to Chemical Formula 4-4: 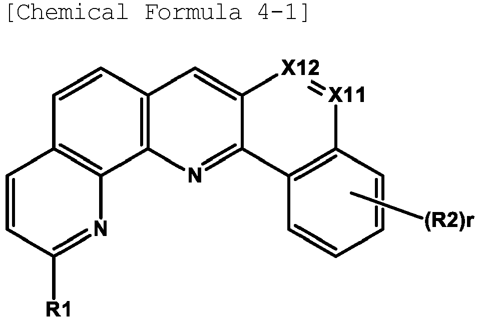 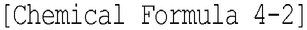 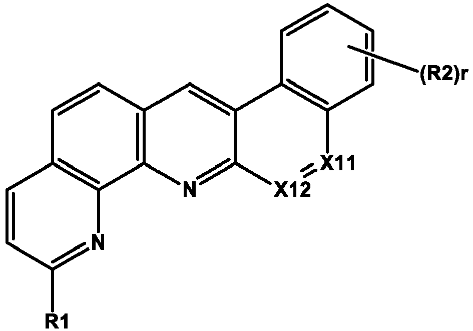 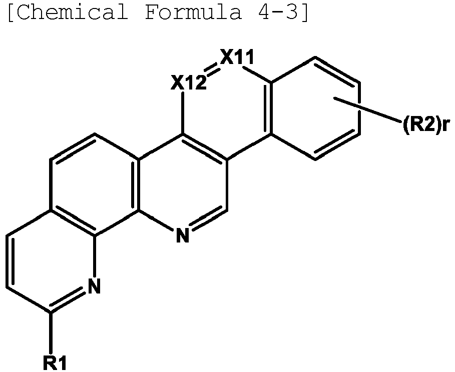 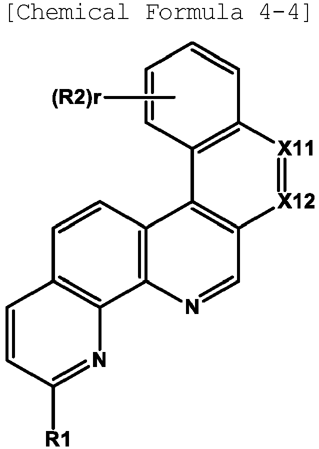
in Chemical Formulae 4-1 to 4-4,
any one of X11 and X12 is N, and the other one is CR; and
R, R1, R2 and r each have the same definition as in Chemical Formula 1.

5. The heterocyclic compound of Claim 1, wherein R and R1 are each independently hydrogen; deuterium; a substituted or unsubstituted phosphine oxide group; a substituted or unsubstituted C6 to C60 aryl group; or a substituted or unsubstituted C2 to C60 heteroaryl group.

6. The heterocyclic compound of Claim 1, wherein R2 is hydrogen; deuterium; a substituted or unsubstituted C6 to C30 aryl group; or a substituted or unsubstituted C2 to C30 heteroaryl group.

7. The heterocyclic compound of Claim 1, wherein Chemical Formula 1 is represented by any one of the following compounds:

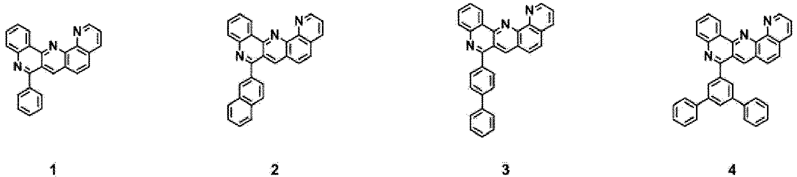

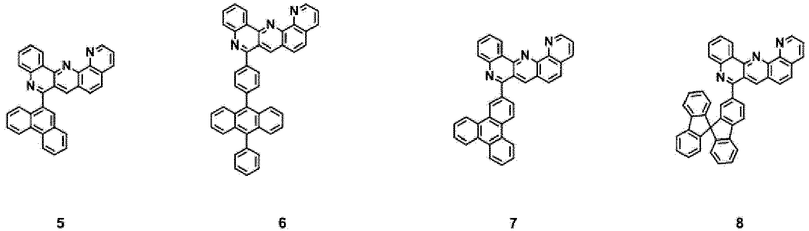

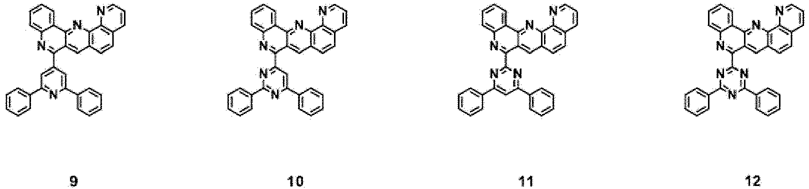

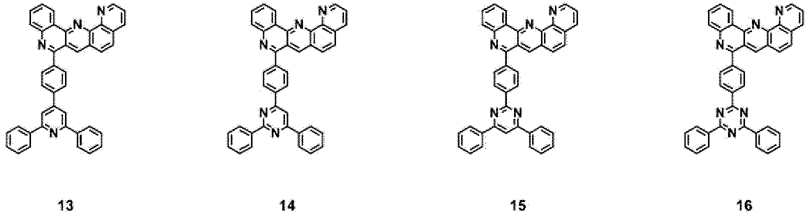

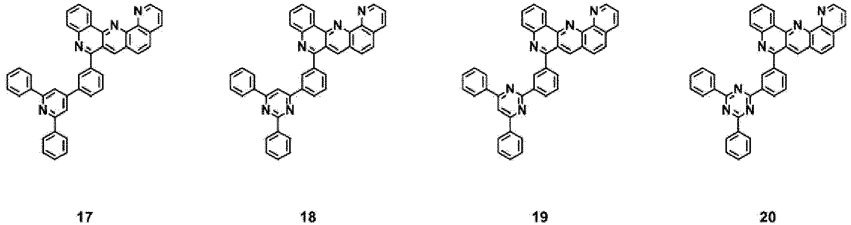

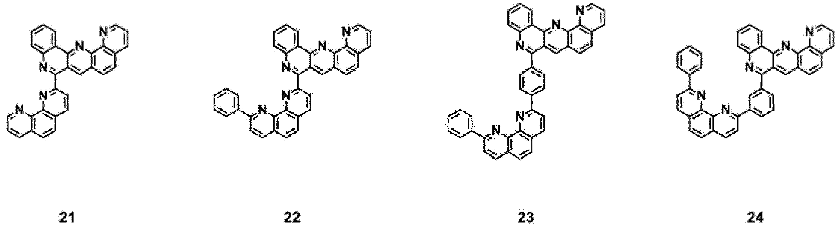

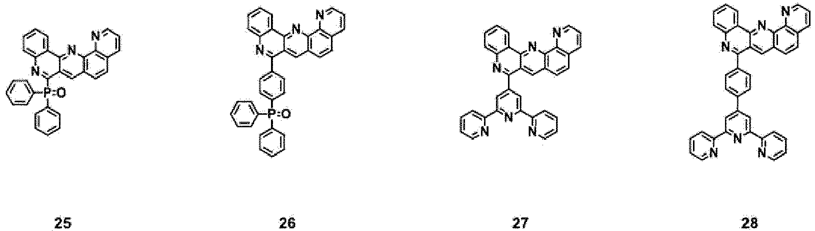

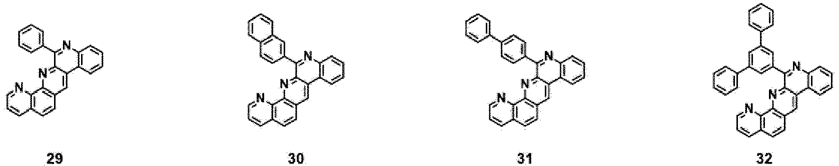

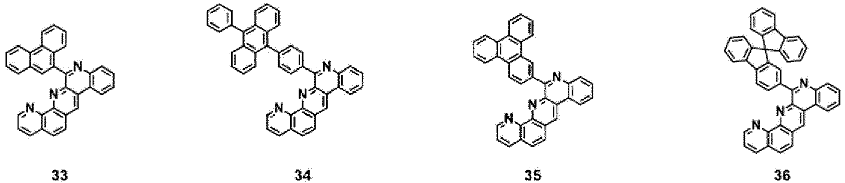

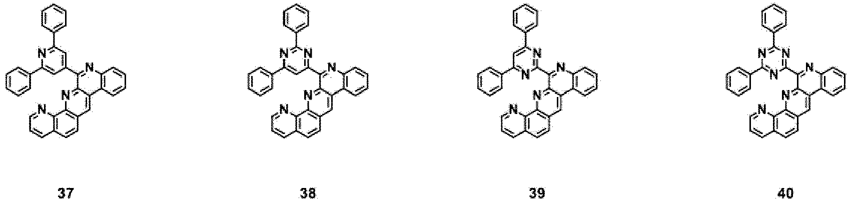

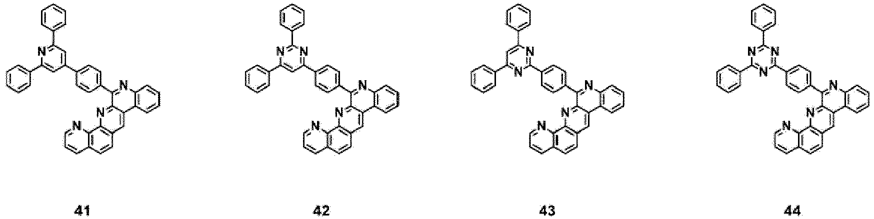

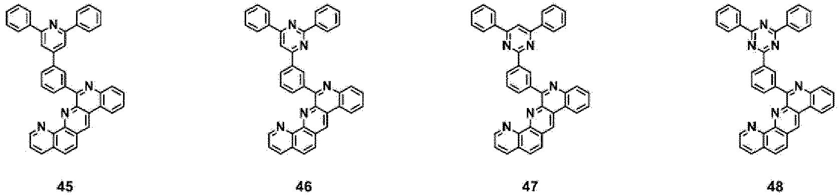

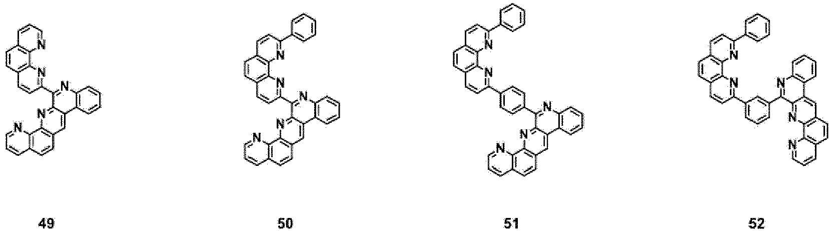

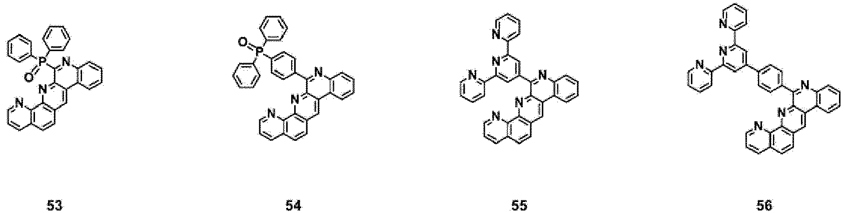

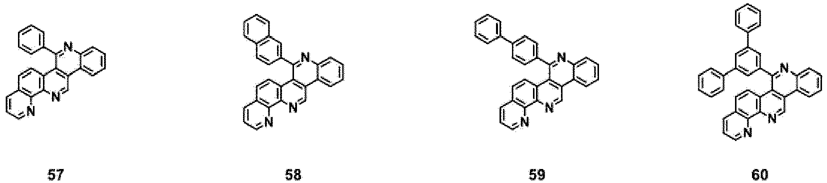

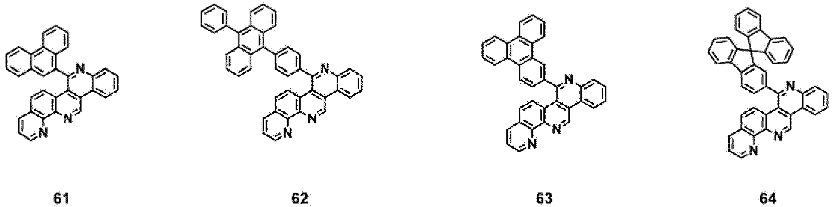

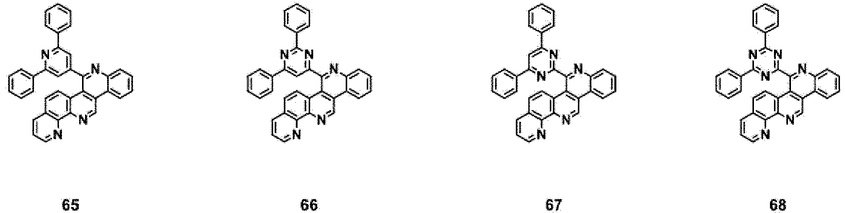

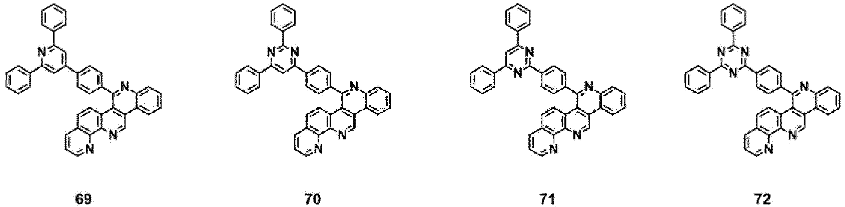

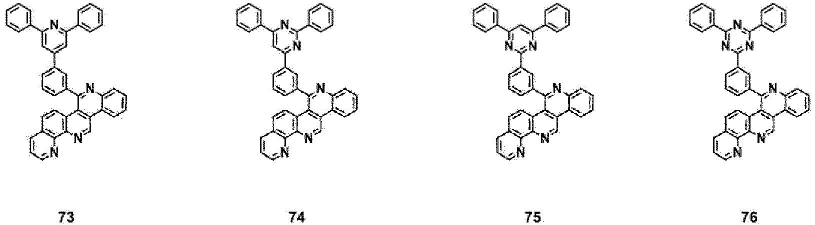

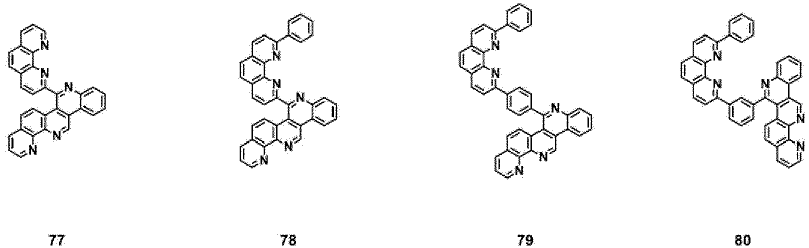

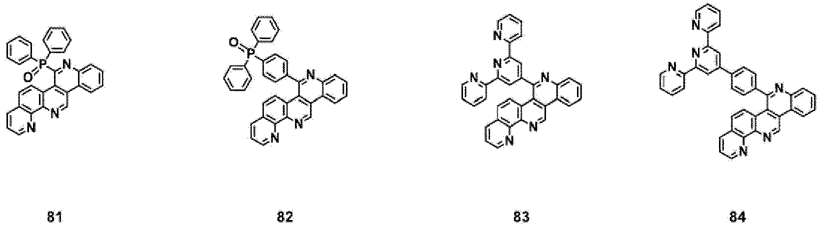

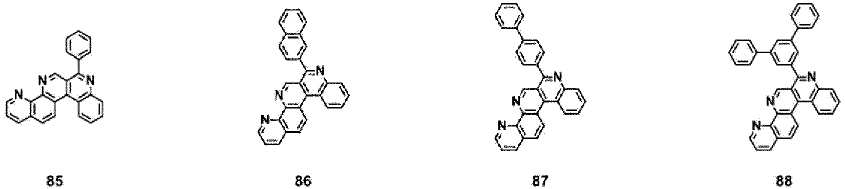

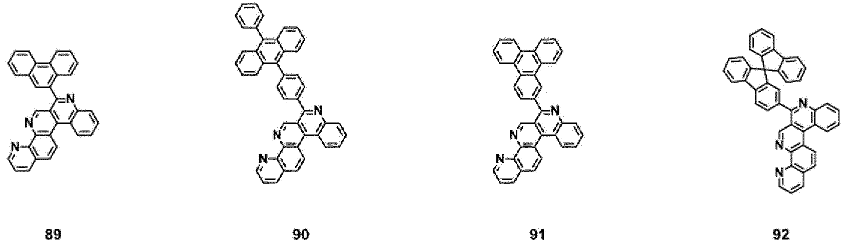

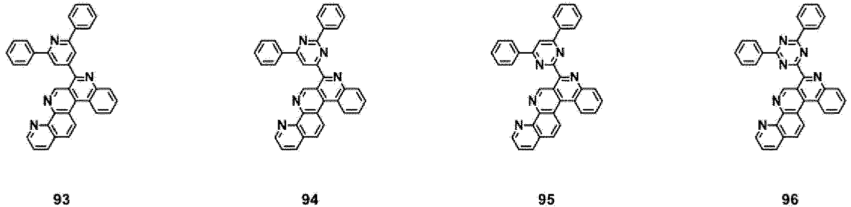

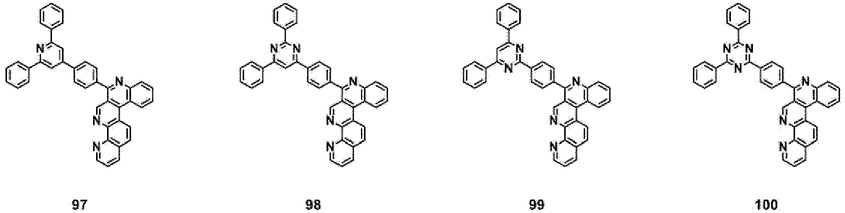

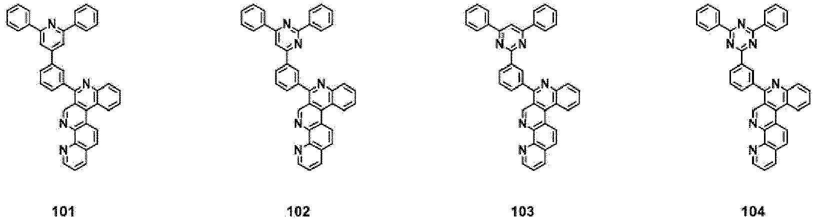

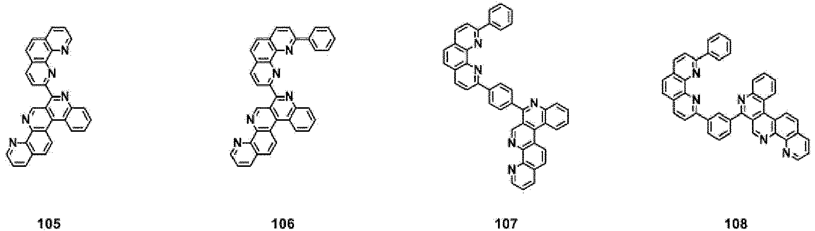

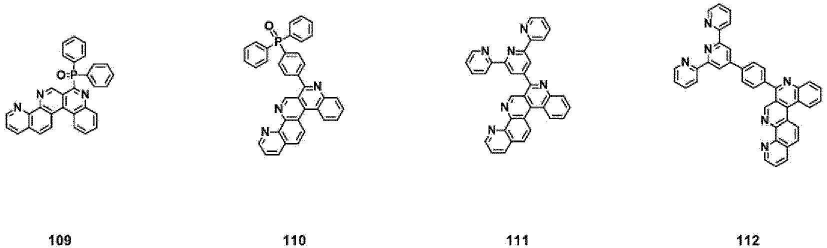

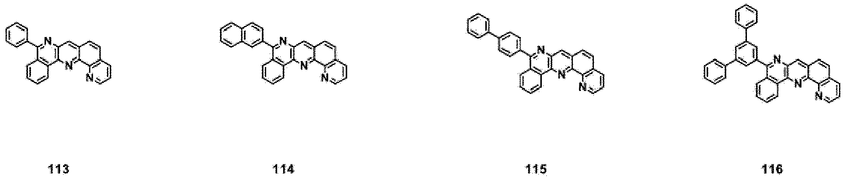

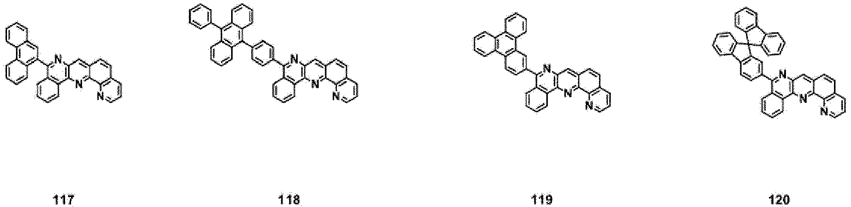

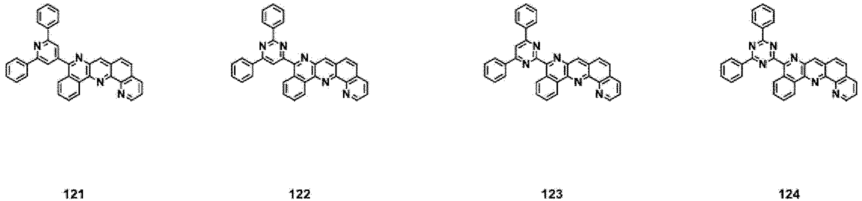

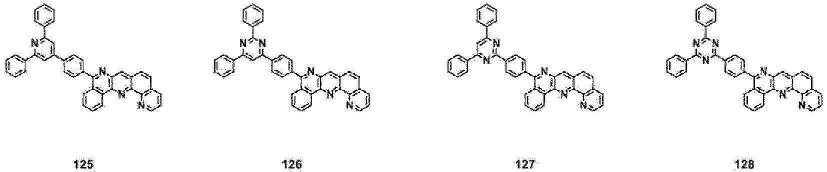

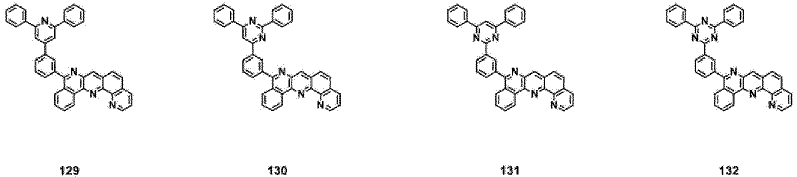

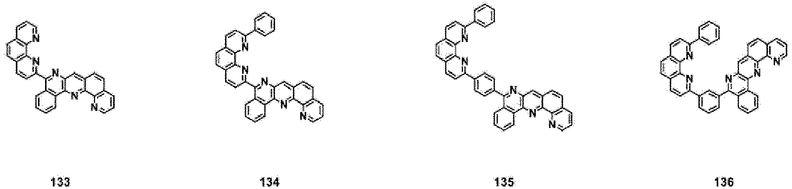

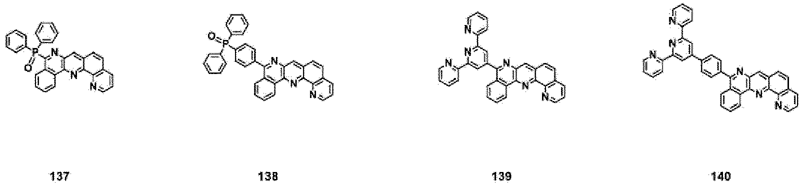

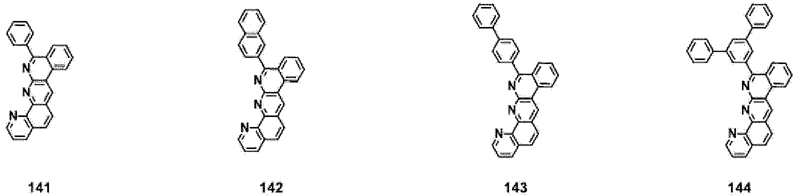

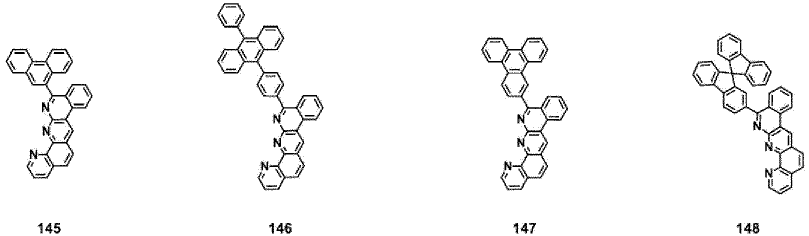

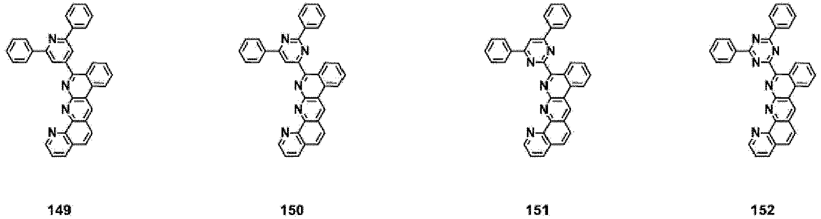

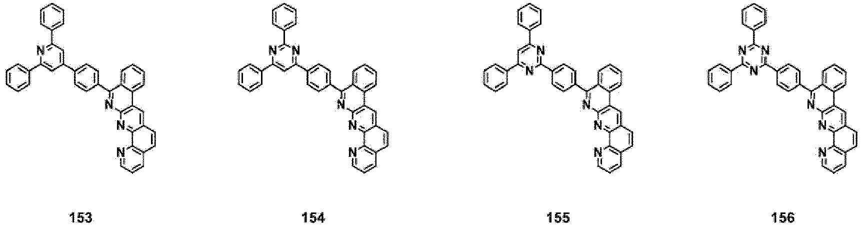

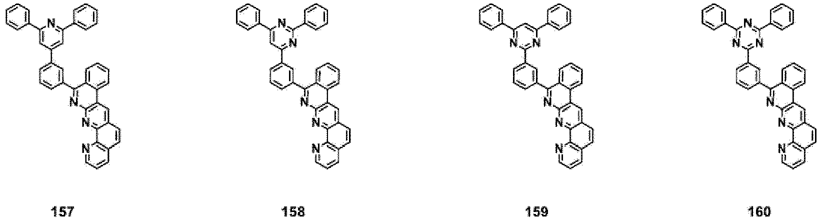

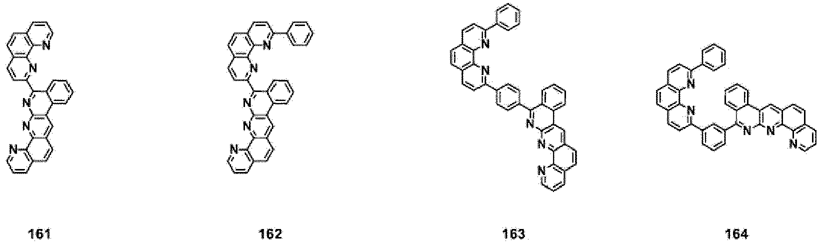

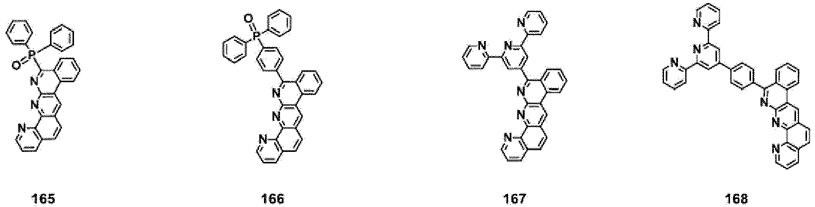

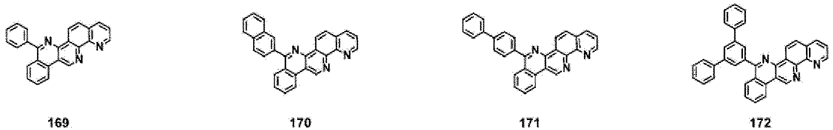

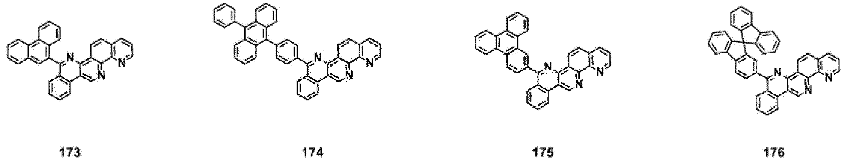

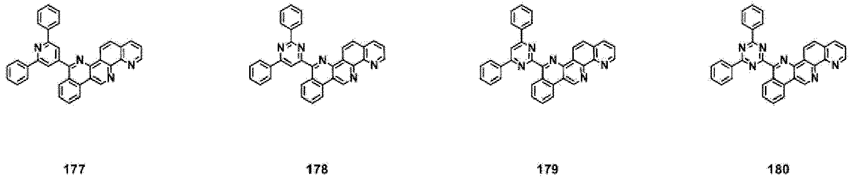

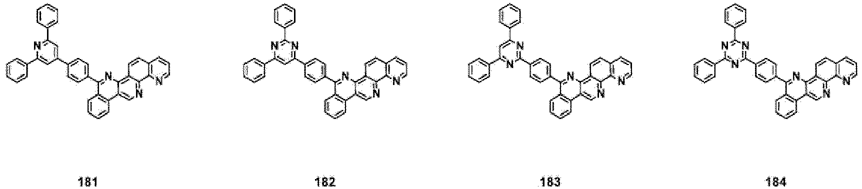

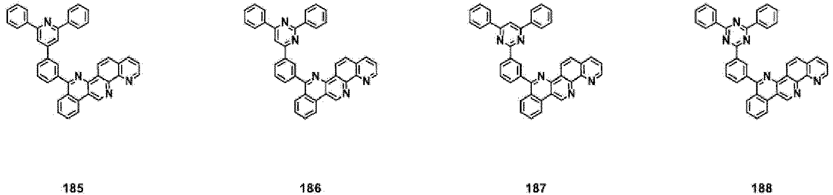

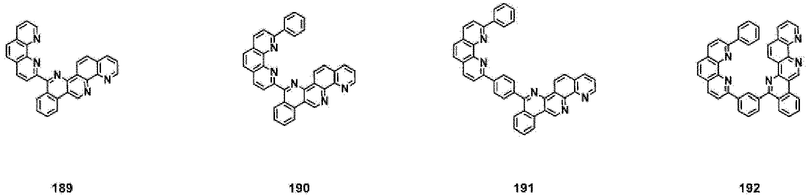

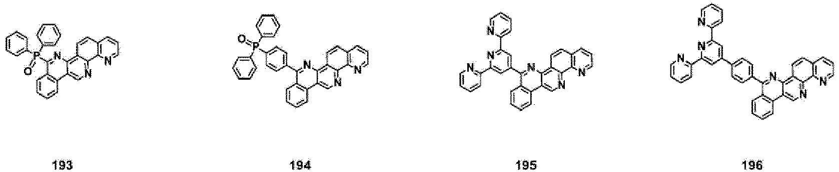

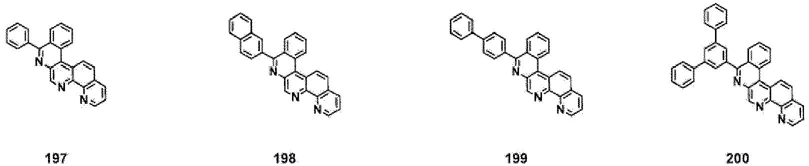

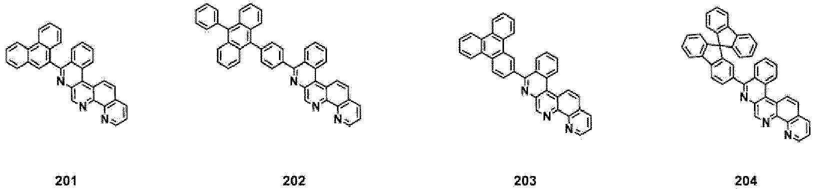

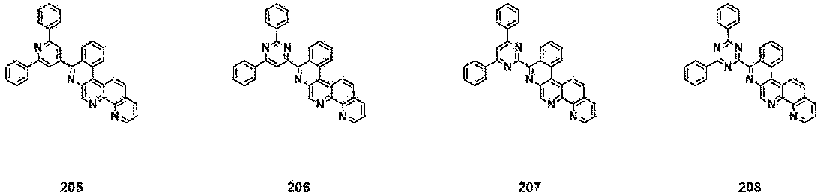

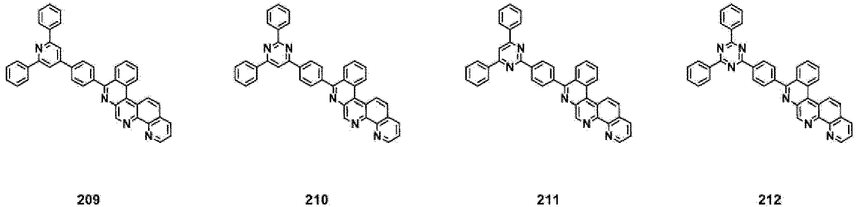

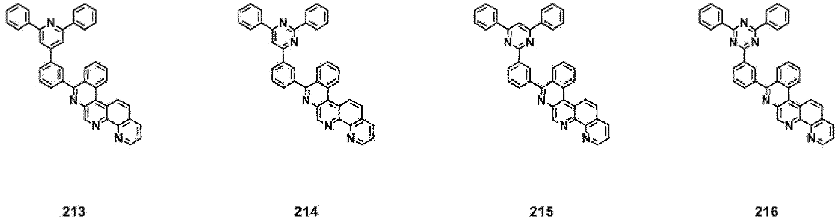

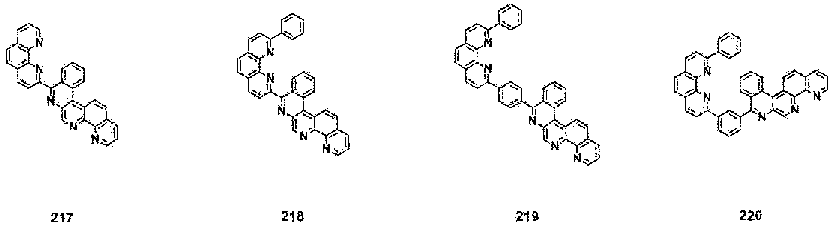

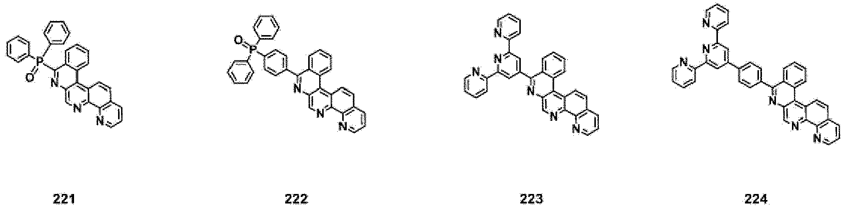

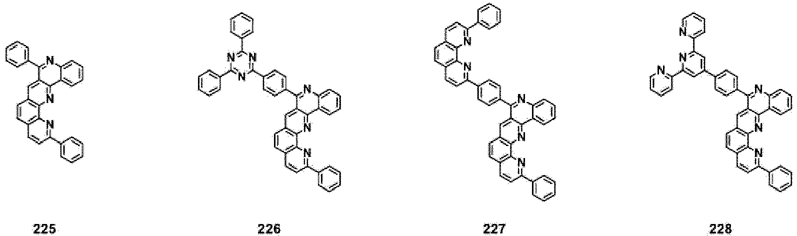

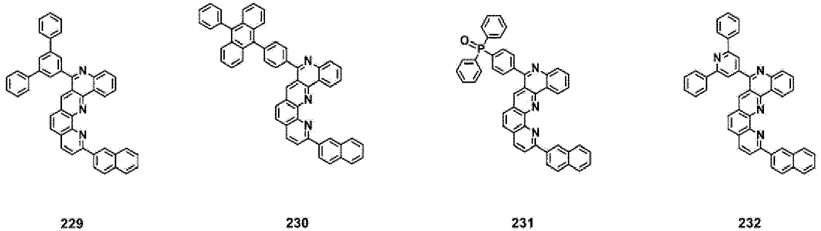

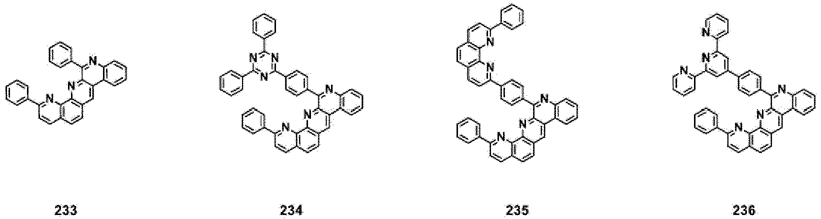

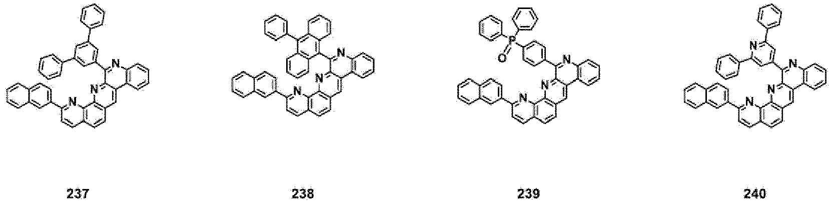

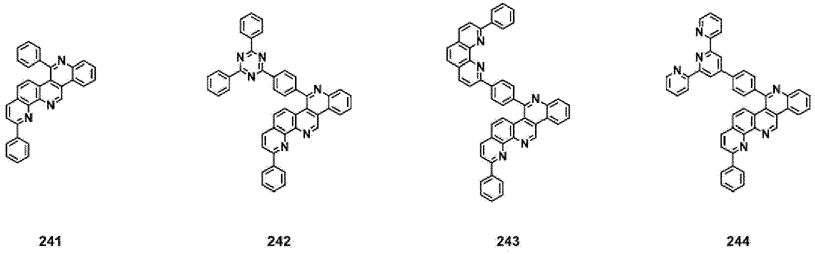

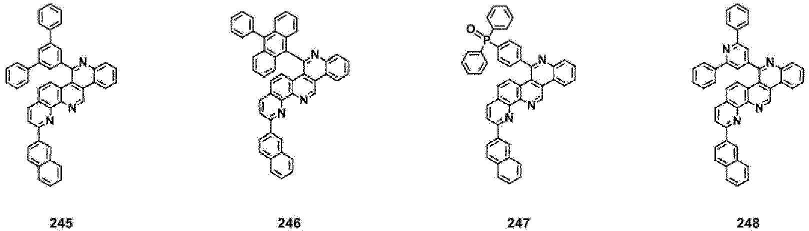

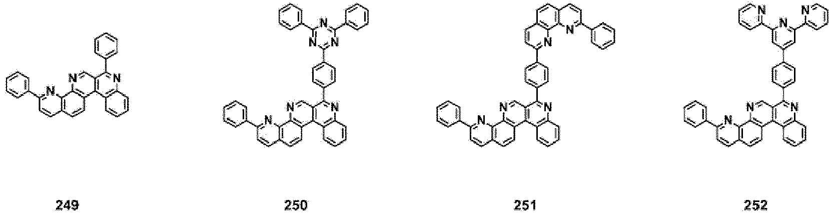

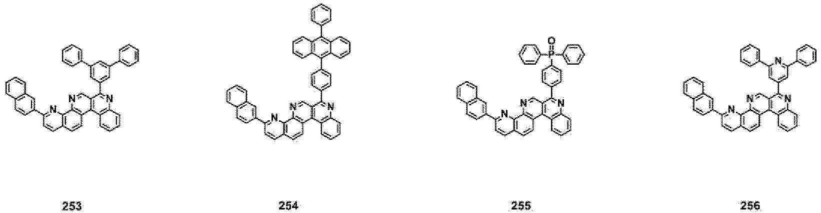

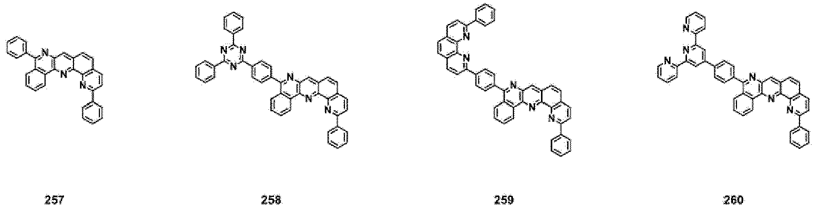

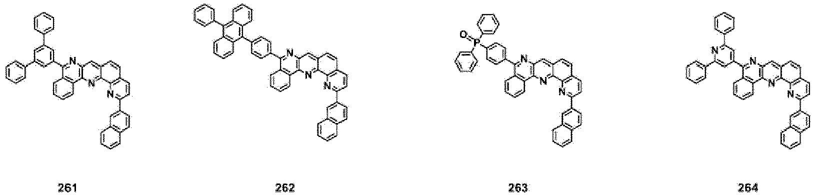

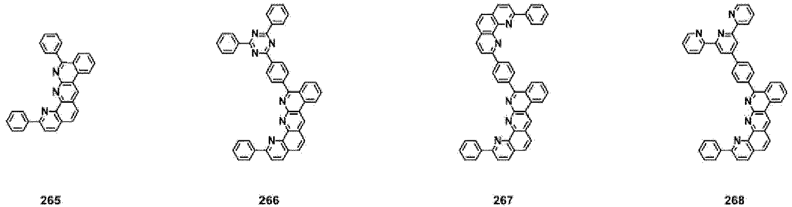

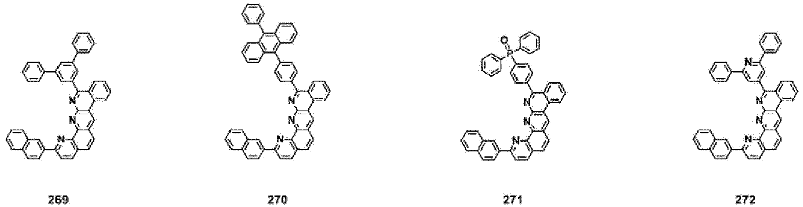

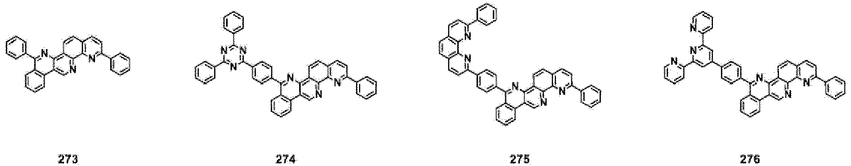

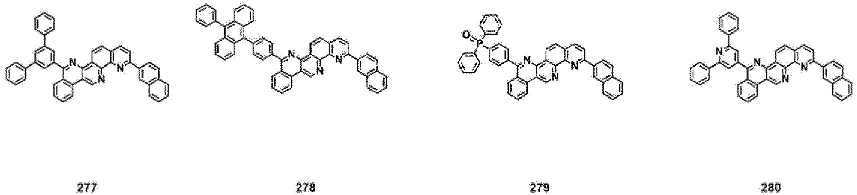

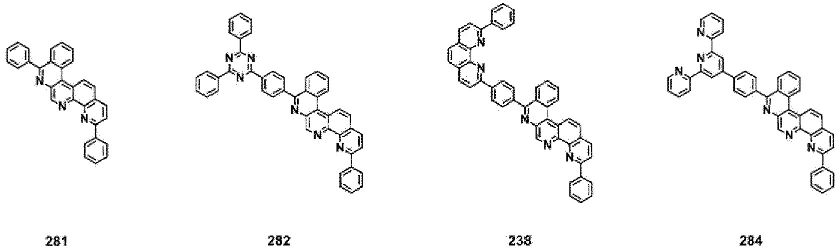

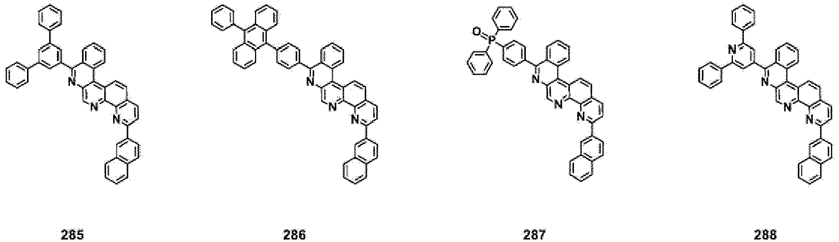

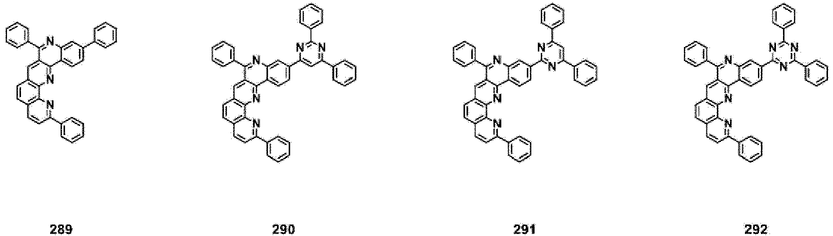

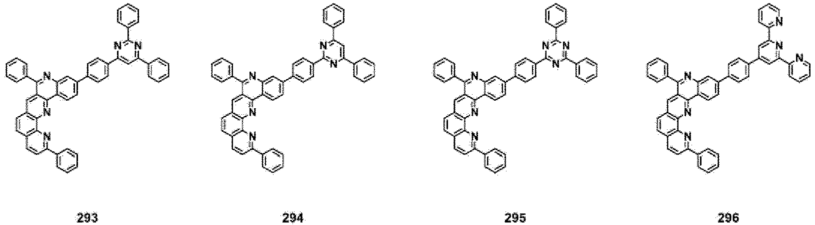

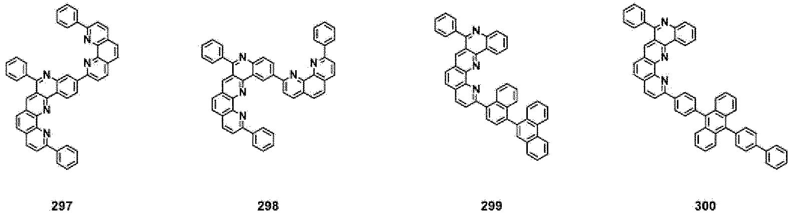

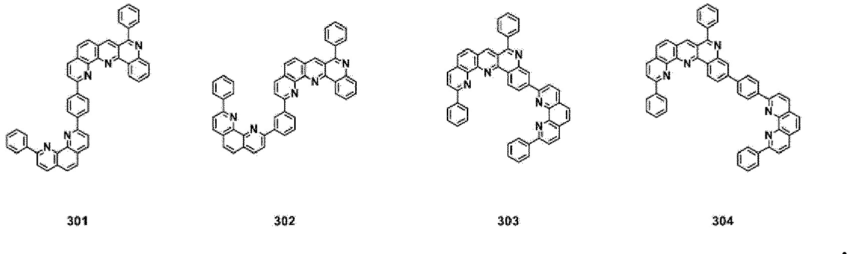

8. An organic light emitting device comprising:
a first electrode;
a second electrode; and
an organic material layer provided between the first electrode and the second electrode,
wherein the organic material layer includes one or more types of the heterocyclic compound of any one of Claims 1 to 7.

9. The organic light emitting device of Claim 8, wherein the organic material layer includes an electron transfer layer, and the electron transfer layer includes the heterocyclic compound.

10. The organic light emitting device of Claim 8, further comprising one layer selected from the group consisting of a light emitting layer, a hole injection layer, a hole transfer layer, an electron injection layer, an electron transfer layer, an electron blocking layer and a hole blocking layer.

11. The organic light emitting device of Claim 8, wherein the organic material layer includes a first stack including a first light emitting layer, a charge generation layer provided on the first stack, and a second stack provided on the charge generation layer and including a second light emitting layer.

12. The organic light emitting device of Claim 11, wherein the charge generation layer includes the heterocyclic compound.

13. The organic light emitting device of Claim 11, wherein the charge generation layer includes an N-type charge generation layer, and the N-type charge generation layer includes the heterocyclic compound.
